# EUROPÄISCHE PATENTSCHRIFT

(11) **EP 2 262 757 B1**
(45) Veröffentlichungstag und Bekanntmachung des Hinweises auf die Patenterteilung: **13.07.2011**
(21) Anmeldenummer: 09716090.7
(22) Anmeldetag: 27.02.2009
(51) Int. Cl.: C07C 213/00, C07C 215/42

(54) **VERFAHREN ZUR HERSTELLUNG VON 6-DIMETHYLAMINOMETHYL-PHENYL-CYCLOHEXAN-1,3-DIOLEN**
METHOD FOR THE PRODUCTION OF 6-DIMETHYL AMINO METHYL-PHENYL-CYCLOHEXANE-1,3-DIOLS
PROCEDE DE FABRICATION DE 6-DIMETHYLAMINOMETHYL-PHENYL-CYCLOHEXANE-1,3-DIOLS

(30) Priorität: 29.02.2008 EP 08003846
(43) Veröffentlichungstag der Anmeldung: 22.12.2010
(73) Patentinhaber: Grünenthal GmbH, 52078 Aachen (DE)
(72) Erfinder: HELL, Wolfgang, 52066 Aachen (DE); ZIMMER, Oswald, 52146 Würselen (DE); KEGEL, Markus, 41239 Mönchengladbach (DE); SCHÄFER, Olaf, 52223 Stolberg (DE); SPINDLER, Felix, 4656 Starrkirch-Wil (CH); SCHNYDER, Anita, 4423 Hersberg (CH); SIEGRIST, Urs, 5070 Frick (CH); DREXLER, Hans-Joachim, 18198 Gross-Schwass (DE); HELLER, Detlef, 18057 Rostock (DE)
(74) Vertreter: Brosch, Oliver
(86) Internationale Anmeldenummer: PCT/EP2009/001394
(87) Internationale Veröffentlichungsnummer: WO 2009/106335

(56) Entgegenhaltungen:
- EP-A- 0 753 506
- EP-A- 0 780 369
- DE-C1- 19 704 401
- GAIS H-J ET AL: "Enzymatic resolution of analgesics: delta-hydroxytramadol, epsilon-hydroxytramadol and O-desmethyltramadol" TETRAHEDRON ASYMMETRY, PERGAMON, OXFORD; GB, Bd. 11, Nr. 4, 1. März 2000 (2000-03-01), Seiten 917-928, XP004192904 ISSN: 0957-4166

## Beschreibung

Die vorliegende Erfindung betrifft ein Verfahren zur Herstellung von 6-Dimethylaminomethyl-1-(3-hydroxy- oder 3-C₁-C₄-alkoxyphenyl)-cyclohexan-1,3-diolen aus 6-Dimethylaminomethyl-1-hydroxy-1-(3-hydroxy- oder 3-C₁-C₄-alkoxyphenyl)-cyclohexan-3-onen mittels katalytischer Hydrierung in Gegenwart heterogener oder homogener Katalysatoren, oder mittels Metallhydriden.

In der EP-A1-0 753 506 werden 6-Dimethylaminomethyl-1-(3-hydroxy- oder 3-C₁-C₄-alkoxyphenyl)-cyclohexan-1,3-diole beschrieben, die als wirksame Analgetika insbesondere zur Behandlung starker Schmerzzustände geeignet sind. Diese Verbindungen besitzen 3 asymmetrische C-Atome. Die 6-Dimethylaminomethyl-1-(3-hydroxy- oder 3-C₁-C₄-alkoxyphenyl)-cyclohexan-1,3-diole werden durch Hydrierung entsprechender racemischer 3-Cyclohexanone mit Metallhydriden wie zum Beispiel Natriumborhydrid (Beispiel 18) oder Diisobutylaluminiumhydrid (Beispiel 20) erhalten und eine Trennung der gebildeten Racemate mittels HPLC an chiralen Säulen führt dann zu den 1 R,3R,6R- und 1 S,3S,6S-Stereoisomeren. Diese Art der Herstellung von enantiomerenreinen Verbindungen ist für industrielle Anwendungen nicht besonders vorteilhaft. Es ist ferner bekannt, dass sich sowohl die erwähnten Cyclohexan-1,3-diole als auch die Ausgangsmaterialien (3-Cyclohexanone) leicht zersetzen, beziehungsweise zu Eliminierungsreaktionen neigen unter Bildung ungesättigter Verbindungen. Auf Grund dieser Empfindlichkeit der Verbindungen kann die Bildung von unerwünschten Nebenprodukten bei einer Hydrierung so hoch sein, oder sogar überwiegen (wie zum Beispiel bei der Reduktion mit Diisobutylaluminiumhydrid), dass diese Reaktionen nicht besonders wirtschaftlich und somit weniger geeignet sind zur industriellen Herstellung von pharmazeutischen Wirkstoffen.

Es ist gezeigt worden, dass sich bestimmte Stereoisomere durch eine besonders gute Wirksamkeit auszeichnen. Bei diesen Stereoisomeren handelt es sich um die 1 R,3R,6R- und 1 S,3S,6S- Stereoisomeren der Formeln III und IV oder deren Gemische

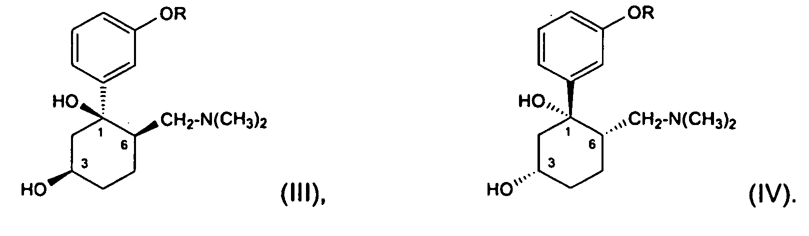

Die 1 R,3S,6R- und 1 S,3R,6S- Stereoisomere der Formeln V und VI 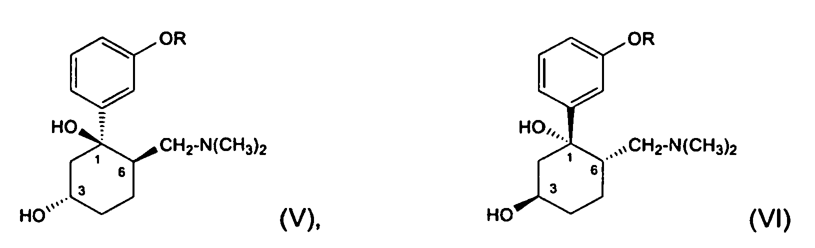 sind dagegen weniger erwünscht und ihre Bildung sollte daher bei Herstellverfahren möglichst unterdrückt werden.

Eine Aufgabe der vorliegenden Erfindung bestand daher darin, ein Verfahren zu einer gezielten Herstellung von Stereoisomeren der Formeln III und IV oder deren Gemischen zur Verfügung zu stellen, bei denen die Bildung von unerwünschten Nebenprodukten oder Zersetzungsprodukten weitgehend unterdrückt ist.

Es wurde nun überraschend gefunden, dass eine gezielte Herstellung von Stereoisomeren der Formeln III und IV oder Mischungen davon möglich ist und bei guten bis hohen Umsätzen und Ausbeuten hervorragende Selektivitäten und Reinheiten erzielt werden können, wenn man die Ketogruppe von 1 R,6R- oder 1 S,6S-6-Dimethylaminomethyl-1-hydroxy-1-(3-hydroxy- oder 3-C₁-C₄-alkoxyphenyl)-cyclohexan-3-on oder Mischungen davon in Gegenwart von ausgewählten Katalysatoren in heterogener oder homogener Phase mit Wasserstoff, oder mit Alkalimetall-trialkylborhydriden oder Alkatimetall-trialkylaluminiumhydriden hydriert. Überraschend kann die Bildung von Nebenprodukten erheblich reduziert werden, zum Beispiel auf unter 10 Gew.-% und sogar unter 5 Gew.-% im Reaktionsprodukt. Wenn man frisch hergestelltes Ausgangsprodukt verwendet, kann der Anteil an Nebenprodukten oft auf 3 Gew.-% oder weniger gesenkt werden.

Gegenstand der Erfindung ist daher ein Verfahren zur Herstellung von Verbindungen der Formeln III oder IV oder Mischungen davon, 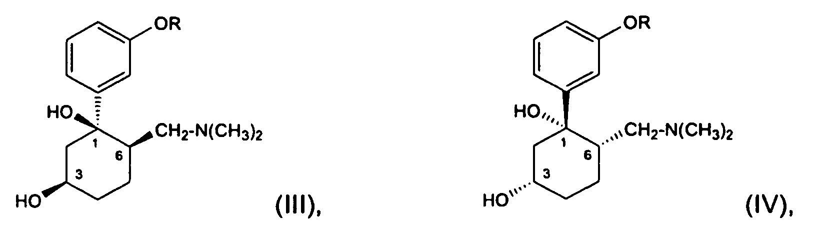 worin R für Wasserstoff oder C₁-C₄-Alkyl steht, durch Hydrierung der Ketogruppe von enantiomerenreinen Verbindungen der Formeln I oder II oder Mischungen davon, 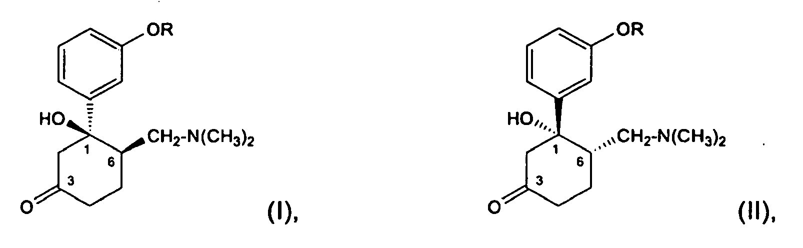 das dadurch gekennzeichnet ist, dass man die Hydrierung
a) mit Wasserstoff in Gegenwart von Platindioxid oder Raney-Nickel in heterogener Phase;
b) mit Wasserstoff in Gegenwart eines Rhodium-, Iridium- oder Rutheniumkomplexes mit chiralen Diphosphin-Liganden in homogener Phase; oder
c) mit einem Alkalimetall-trialkylborhydrid oder einem Alkalimetalltrialkylaluminiumhydrid durchführt.

Bei R als Alkyl kann es sich zum Beispiel um Methyl, Ethyl, n- und iso-Propyl, oder n-, iso-, sec- und tert-Butyl handeln. In einer bevorzugten Ausführungsform stellt R Wasserstoff oder insbesondere Methyl dar. In einer weiteren bevorzugten Ausführungsform ist R Methyl.

Nach dem erfindungsgemäßen Verfahren werden die Diastereomeren der Formeln III und IV überwiegend diastereomerenrein erhalten, d.h., dass das Verhältnis von Diastereomeren der Formeln III und IV zu Diastereomeren der Formeln V und VI (im folgenden auch als Selektivität bezeichnet) beispielsweise mindestens 75:25, bevorzugt mindestens 80:20, bevorzugter mindestens 85:15 und besonders bevorzugt mindestens 90:10 beträgt. Abhängig von der Wahl der Katalysatoren und Reaktionsbedingungen kann bei der heterogenen und homogenen Katalyse sogar ein Verhältnis von mindestens 95:5 und höher erzielt werden. Die Umsetzung mit Alkalimetall-trialkylborhydriden oder Alkalimetall-trialkylaluminiumhydriden führt sogar zu Verhältnissen von wenigstens 99:1.

Das erfindungsgemäße Verfahren kann insbesondere bei Normaldruck oder Überdruck durchgeführt werden. Bei Normaldruck und geringem Überdruck werden häufig bessere Selektivitäten beobachtet. Der Wasserstoffdruck kann bei den Verfahrensvarianten a) und b) zum Beispiel von 10⁵ bis 2x10⁷ Pa (Pascal) betragen. Bei Verfahrensvariante a) wird bevorzugt ein Wasserstoffdruck von 10⁵ bis 10⁷ Pa, vorzugsweise von 10⁵ bis 5x10⁶ Pa und bei Verfahrensvariante b) ein Wasserstoffdruck von 10⁵ bis 10⁷ Pa, vorzugsweise von 10⁶ bis 10⁷ Pa angewendet. Das erfindungsgemäße Verfahren kann bei tiefen oder erhöhten Temperaturen, zum Beispiel Temperaturen von -80 bis 150 °C, bevorzugt von -20 bis 100 °C, und besonders bevorzugt von -20 bis 80 °C durchgeführt werden. Die optischen Reinheiten sind im allgemeinen bei tieferer Temperatur besser als bei höheren Temperaturen. Die Reaktionsgeschwindigkeiten und Umsätze sind dagegen bei tieferen Temperaturen niedriger. Die Verfahrensvariante c) wird vorteilhaft bei Temperaturen von -100 bis 20°C, bevorzugt bei -90 bis 10 °C und bevorzugter bei - 80 bis 0 °C durchgeführt.

Platindioxid, ein käuflicher Katalysator, wird bevorzugt in Mengen von 0,1 bis 15 Gew.-%, besonders bevorzugt 0,5 bis 12,5 Gew.-%, und insbesondere bevorzugt 1 bis 10 Gew.-% verwendet, bezogen auf die Menge der Verbindungen der Formeln I und II. Raney-Nickel, ebenfalls ein käuflicher Katalysator, wird bevorzugt in Mengen von 1 bis 50 Gew.-%, besonders bevorzugt 3 bis 50 Gew.-%, und insbesondere bevorzugt 5 bis 40 Gew.-% verwendet, bezogen auf die Menge der Verbindungen der Formeln I und II. Die Rhodium-, Iridium- oder Rutheniumkomplexe mit chiralen Diphosphinen werden bevorzugt in Mengen von 0,0001 bis 10 Gew.-%, besonders bevorzugt 0,001 bis 5 Gew.-%, und insbesondere bevorzugt 0,01 bis 3 Gew.-% verwendet, bezogen auf die Menge der Verbindungen der Formeln I und II. Die Alkalimetall-trialkylborhydride und Alkalimetall-trialkylaluminiumhydride werden in der Regel in äquivalenten Mengen oder einem geringen Überschuss von zum Beispiel bis zu 0,5 Äquivalenten beziehungsweise Unterschuss von bis zu 0,2 Äquivalenten eingesetzt, bezogen auf die Menge der Verbindungen der Formeln I und II.

Heterogene Katalysatoren können rezykliert werden, wobei ein teilweiser Ersatz mit frischem Katalysator Aktivitätsverluste ausgleichen kann.

Das erfindungsgemäße Verfahren und gegebenenfalls eine in situ Herstellung von homogenen Katalysatoren kann ohne oder in Gegenwart eines vorzugsweise inerten Lösungsmittels (Reaktionsmediums) durchgeführt werden, wobei ein Lösungsmittel oder Gemische von zwei oder mehr, beispielsweise zwei oder drei, Lösungsmitteln eingesetzt werden können. Geeignete Lösungsmittel sind zum Beispiel aliphatische, cycloaliphatische und aromatische Kohlenwasserstoffe (z.B. Pentan, Hexan, Petrolether, Cyclohexan, Methylcyclohexan, Benzol, Toluol, Xylol), aliphatische Halogenkohlenwasserstoffe (z.B. Methylenchlorid, Chloroform, Di- und Tetrachlorethan), Nitrile (z.B. Acetonitril, Propionitril, Benzonitril), Ether (z.B. Diethylether, Diisopropylether, Dibutylether, t-Butylmethylether, Ethylenglykoldimethylether, Ethylenglykoldiethylether, Diethylenglykoldimethylether, Tetrahydrofuran, Dioxan, Diethylenglykolmonomethyl- oder monoethylether), Carbonsäureester und Lactone (z.B. Essigsäureethyl- oder -methylester, Valerolacton), N-substituierte Lactame (z.B. N-Methylpyrrolidon), Carbonsäureamide (z.B. Dimethylformamid, Dimethylacetamid), acyclische Harnstoffe (z.B. Dimethylimidazolin), Sulfoxide und Sulfone (z.B. Dimethylsulfoxid, Dimethylsulfon, Tetramethylensulfoxid, Tetramethylensulfon), Alkohole (z.B. Methanol, Ethanol, Propanol, Butanol, Ethylenglykolmonomethylether, Ethylenglykolmonoethylether, Diethylenglykolmonomethylether) und Wasser. Die Lösungsmittel können alleine oder in Mischung von wenigstens zwei Lösungsmitteln verwendet werden. Basische Lösungsmittel sind weniger bevorzugt, da sie die Bildung von Nebenprodukten fördern können.

Bei der Verwendung von Platindioxid können bevorzugt Zusätze wie Eisessig oder Metallhalogenide (z.B. MgCl₂) in Mengen von zum Beispiel 0,5 bis 1,5 Äquivalenten, bezogen auf die Menge der Verbindungen der Formeln I und II, eingesetzt werden. Die Hydrierung mit Platindioxid wird bevorzugt in Alkoholen (z.B. Methanol, Ethanol, Propanol und Butanol) oder in Essigsäure (z.B. Eisessig) als Lösungsmittel durchgeführt.

Die Hydrierung mit Raney-Nickel wird bevorzugt in Alkoholen (z.B. Methanol, Ethanol, Propanol und Butanol), Carbonsäureestern (z.B. Essigsäureethylester) oder aromatischen Kohlenwasserstoffen (z.B. Toluol, Xylol) als Lösungsmittel durchgeführt.

Die Verbindungen der Formeln I oder II können in solchen Mengen eingesetzt werden, dass Lösungen oder Suspensionen vorliegen.

Für das erfindungsgemäße Verfahren der Hydrierung der Verbindungen der Formeln I und II mit Wasserstoff mittels homogener Katalyse sind Metallkomplexe des Rhodiums, Iridiums und bevorzugt Rutheniums mit Diphosphin-Liganden geeignet, die ggf. in-situ gebildet werden können.

Als Diphosphin-Liganden kommen zum Beispiel Diphosphine und Analoge in Frage, wie sie zum Beispiel in Übersichten zu finden sind, unter anderem in a) H. Brunner, W. Zettlmeier, Handbook of Enantioselective Catalysis, VCH Weinheim, 1993, Vol. 2, Seite 3ff.; b) R. Noyori, et al. in Catalytic Asymmetric Synthesis Second Edition (I. Ojima, Ed.), Wiley-VCH, Weinheim, 2000, Seite 1ff.; c) E.N. Jacobsen, A. Pfaltz, H. Yamamoto (Eds.), Comprehensive Asymmetric Catalysis Vol I-III, Springer Berlin, 1999, und darin angegebene Referenzen.

Möglich sind generell chirale Strukturen der Art Sekundärphosphin-Gerüst-Sekundärphosphin. Die zwei Sekundärphosphingruppen sind bevorzugt so an ein Gerüst gebunden, dass im Metallkomplex zusammen mit dem Metallatom ein 5- bis 1 0-gliedriger und bevorzugter 5- bis 8-gliedriger Ring gebildet wird. Die zwei Sekundärphosphingruppen sind ggf. endständig an den C-Atomen einer C₂-C₈-, bevorzugt C₂-C₆-, und besonders bevorzugt C₂-C₄-Kette gebunden, wobei C-Atome der Kette durch Heteroatome O, S, NH und/oder N-C₁-C₄-Alkyl ersetzt und die Kohlenstoffkette Teil eines monozyklischen oder polyzyklischen Ringes sein können. Das Gerüst kann vorzugsweise 2 bis 30, bevorzugter 2 bis 20 C-Atome und gegebenenfalls zusätzlich Heteroatome enthalten, vorzugsweise 1, 2, 3 oder 4 Heteroatome. Das Gerüst kann ferner Atome der Übergansmetalle aufweisen, wie z.B. Eisen. Die Heteroatome können bevorzugt ausgewählt sein aus der Gruppe bestehend aus O, S, NH und N-C₁-C₄-Alkyl. Das Gerüst kann unsubstituiert oder einfach oder mehrfach, beispielsweise 1-, 2-, 3-, 4-, 5- oder 6-fach substituiert sein, zum Beispiel mit Substituenten unabhängig voneinander ausgewählt aus der Gruppe bestehend aus C₁-C₆-Alkyl, C₁-C₆-Alkoxy, C₁-C₆-Alkylthio, C₄-C₈-Cycloalkyl, Phenyl, Benzyl, Phenoxy, Benzyloxy, Phenylthio, Benzylthio, Halogen (bevorzugt F, Cl, Br), OH, Tri(C₁-C₆-Alkyl)silyl, Sekundäramino, -CO₂H, -SO₃H, -CO₂R', -SO₃R', -O-C(O)-R', -NH-C(O)R', -O-SO₃-R', und -NH-SO₃R', wobei R' für C₁-C₆-Alkyl, C₄-C₈-Cycloalkyl, Phenyl oder Benzyl steht. Bei dem Gerüst kann es sich insbesondere um bivalente Reste von Alkanen, Heteroalkanen, Alkenen, Zykloalkanen, Zykloalkenen, Heterozykloalkanen, Heterozykloalkenen, Bizykloalkanen, Bizykloheteroalkanen, Spirobiszykloalkanen, Spirobiszykloheteroalkanen, Arylenen, Heteroarylenen, Bisarylenen, Bisheteroarylenen oder Metallocenen wie zum Beispiel Ferrocenen, handeln, wobei eine oder beide Phosphingruppen über eine Methylen, C₁-C₁₂-Alkyliden, Phenylen oder -CR"R*-Phenylen an den Cyclopentadienylring eines Metallocens gebunden sein können. R" und R* sind unabhängig voneinander zum Beispiel C₁-C₆-Alkyl, C₁-C₆-Alkoxy, oder Phenyl. Die freien Bindungen befinden sich an einem oder beiden Cyclopentadienylringen. In den zyklischen Gerüsten befinden sich die freien Bindungen bevorzugt in 1,2-Stellungen und in 1,1'-Bisarylen in 6,6'-Stellung. Bevorzugt können Liganden können eingesetzt werden, die C2-Symmetrie aufweisen.

Die Sekundärphosphingruppen können auch über ein Sauerstoffatom an C-Atome des Gerüstes gebunden sein (es handelt sich dann um Phosphinite).

Eine sekundäre Phosphingruppe kann durch einen Oxazolinylrest der Formel 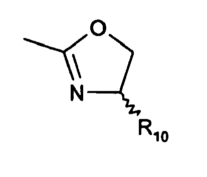 ersetzt werden, besonders in Ferrocengerüsten, wobei R₁₀ C₁-C₆-Alkyl, Cyclohexyl, Phenyl oder Benzyl bedeutet.

Die Chiralität der Diphosphinliganden kann insbesondere auf einer planaren Isomerie (z.B. bei Ferrocenen), Atropisomerie, der Anwesenheit asymmetrischer C-Atome und/oder P-Atome oder beliebigen Kombinationen davon beruhen.

Beispiele für Gerüste von Atropisomeren sind 1,1'-Bisaryle und -Bisheteroaryle (wie zum Beispiel Biphenyl, Binaphthyl oder Bisthiophenyl) mit in den 2,2'-Stellungen gebundenen sekundären Phosphingruppen und gegebenenfalls weiteren Substituenten besonders in der 6- oder in den 6,6'-Stellungen. Dem Fachmann sind entsprechende Liganden beispielsweise unter den Trivialnamen Binap, Biphemp, Biphep und Solphos bekannt. Bekannt sind dem Fachmann ferner auch Bicyclopentane als Grundgerüst, die unter dem Trivialnamen Bicp käuflich erhältlich sind.

Beispiele für Gerüste mit planarer Chiralität sind solche auf Basis von Ferrocenen mit zwei direkt an je einen der Cyclopentadienylringe gebundenen oder an einen Cyclopentadienylring in 1,2-Stellung gebundenen sekundären Phosphingruppen und gegebenenfalls chiralen Substituenten an einem oder beiden Cyclopentadienylringen. Ein anderes Beispiel sind Ferrocene, an die in 1,2-Stellung des Cyclopentadienylringes eine sekundäre Phosphingruppe und eine weitere sekundäre Phosphingruppe über ein asymmetrisches C-Atom gebunden ist. Ein weiteres Beispiel sind Ferrocene, an die in 1,2-Stellung des Cyclopentadienylringes eine sekundäre Phosphingruppe über ein asymmetrisches C-Atom und eine zweite sekundäre Phosphingruppe über 1,2-Phenylen gebunden ist. Beispiele für entsprechende Liganden sind dem Fachmann unter den Trivialnamen Josiphos, Walphos, Taniaphos, Mandyphos und Ferriphos bekannt.

Bekannt sind auch Diphosphine mit chiralen P-Ringen, die besonders in einer oder beiden α-Stellungen zum P-Atom substituiert sind, zum Beispiel Phospholane und Phosphetane. Solche sekundären Phoshingruppen können in 1,2-Stellung von Benzol, Naphthalin, Thiophen, Benzothiophen, Ethan und Ferrocen gebunden sein. Beispiele für entsprechende Liganden sind dem Fachmann unter den Trivialnamen Rophos, Butiphane und Kephos bekannt.

Beispiele für Gerüste mit asymmetrischen C-Atomen sind offenkettige mit in 1,2-, 1,3-oder 1,4-Stellungen, insbesondere aliphatische bizyklische Ringsysteme mit in den 1,2-Stellungen gebundenen sekundären Phosphingruppen, oder zyklische oder heterozyklische Fünfringe mit in 3,4-Stellungen gegebenenfalls über eine Methylengruppe gebundenen sekundären Phosphingruppen. Bekannt sind auch Fünfringe mit in 4-Stellung gebundener sekundärer Phosphingruppe und in 2-Stellung gebundener sekundärer Phosphinmethylgruppe. Trivialnamen für solche Liganden sind Diop, Bppm, Bzppm, Depyphos, Norphos und Prophos.

Beispiele für Diphosphine mit chiralen P-Atomen sind 1,2-Bis(sekundäphosphin)ethane mit unterschiedlichen Substituenten in den Phosphingruppen. Ein bekannter Vertreter ist unter dem Trivialnamen Dipamp erhältlich.

Die sekundären Phosphingruppen können gleiche oder verschiedene Kohlenwasserstoffreste als Substituenten enthalten und die beiden sekundären Phosphingruppen in den Diphosphinen können gleich oder verschieden sein. Häufig können gute Ergebnisse erzielt werden, wenn die sekundären Phosphingruppen nicht identisch, sondern verschieden sind.

Die Kohlenwasserstoffreste können unsubstituiert oder einfach oder mehrfach, beispielsweise 1-, 2-, 3-, 4-, 5- oder 6-fach substituiert sein und/oder beispielsweise 1, 2, 3 oder 4 Heteroatome unabhängig voneinander ausgewählt aus der Gruppe bestehend aus O, S, -N= und N(C₁-C₄-Alkyl) enthalten. Sie können vorzugsweise 1 bis 22, bevorzugter 1 bis 12, und besonders bevorzugt 1 bis 8 C-Atome enthalten.

Ein bevorzugtes Sekundärphosphin ist jenes, worin die Phosphingruppe zwei gleiche oder verschiedene Reste, ausgewählt aus der Gruppe lineares oder verzweigtes C₁-C₁₂-Alkyl, unsubstituiertes oder mit C₁-C₆-Alkyl oder C₁-C₆-Alkoxy substituiertes C₅-C₁₂-Cycloalkyl oder C₅-C₁₂-Cycloalkyl-CH₂-; Phenyl, Naphthyl, Furyl oder Benzyl; oder mit Halogen, C₁-C₆-Alkyl, Trifluormethyl, C₁-C₆-Alkoxy, Trifluormethoxy, (C₆H₅)₃Si, (C₁-C₁₂-Alkyl)₃Si, oder Sekundäramino substituiertes Phenyl oder Benzyl, enthält.

Beispiele für P-Substituenten als Alkyl, das bevorzugt 1 bis 6 C-Atome enthält, sind Methyl, Ethyl, n-Propyl, iso-Propyl, n-Butyl, iso-Butyl, sec-Butyl, tert-Butyl, und die Isomeren von Pentyl und Hexyl. Beispiele für P-Substituenten als gegebenenfalls mit Alkyl substituiertes Cycloalkyl sind Cyclopentyl, Cyclohexyl, Methyl- und Ethylcyclohexyl, und Dimethylcyclohexyl. Beispiele für P-Substituenten als mit Alkyl, und Alkoxy substituiertes Phenyl und Benzyl sind Methylphenyl, Dimethylphenyl, Trimethylphenyl, Ethylphenyl, Methylbenzyl, Methoxyphenyl, Dimethoxyphenyl, Trimethoxyphenyl, Trifluormethylphenyl, Bis-trifluormethylphenyl, Tris-trifluormethylphenyl, Trifluormethoxyphenyl, Bis-trifluormethoxyphenyl, Fluor- und Chlorphenyl und 3,5-Dimethyl-4-methoxyphenyl.

Bevorzugte sekundäre Phosphingruppen sind solche, die gleiche oder verschiedene Reste ausgewählt aus der Gruppe C₁-C₆-Alkyl, unsubstituiertes oder mit 1 bis 3 C₁-C₄-Alkyl oder C₁-C₄-Alkoxy substituiertes Cyclopentyl oder Cyclohexyl, Benzyl und besonders Phenyl, die unsubstituiert oder einfach oder mehrfach substituiert sind mit 1 bis 3 Substituenten unabhängig voneinander ausgewählt aus der Gruppe bestehend aus C₁-C₄-Alkyl, C₁-C₄-Alkoxy, C₁-C₄-Fluoralkyl,C₁-C₄-Fluoralkoxy, F und Cl.

Die Sekundärphosphinogruppe entspricht bevorzugt der Formel -PR₁ R₂, worin R₁ und R₂ unabhängig voneinander einen Kohlenwasserstoffrest mit 1 bis 18 C-Atomen darstellen, der unsubstituiert oder einfach oder mehrfach, beispielsweise 1-, 2-, 3-, 4-, 5- oder 6-fach substituiert ist mit Substituenten unabhängig voneinander ausgewählt aus der Gruppe bestehend aus C₁-C₆-Alkyl, Trifluormethyl, C₁-C₆-Alkoxy, Trifluormethoxy, (C₁-C₄-Alkyl)₂amino, (C₆H₅)₃Si, (C₁-C₁₂-Alkyl)₃Si und Halogen und ggf. eines oder mehrere, beispielsweise 1-4, Heteroatome O enthält.

Bevorzugt sind R₁ und R₂ Reste, ausgewählt aus der Gruppe lineares oder verzweigtes C₁-C₆-Alkyl, unsubstituiertes oder mit ein bis drei C₁-C₄-Alkyl oder C₁-C₄-Alkoxy substituiertes Cyclopentyl oder Cyclohexyl, Furyl, unsubstituiertes oder mit ein bis drei C₁-C₄-Alkyl oder C₁-C₄-Alkoxy substituiertes Benzyl, und insbesondere unsubstituiertes oder mit ein bis drei F, Cl, C₁-C₄-Alkyl, C₁-C₄-Alkoxy, C₁-C₄-Fluoralkyl oder C₁-C₄-Fluoralkoxy substituiertes Phenyl.

Besonders bevorzugt bedeuten R₁ und R₂ Reste, ausgewählt aus der Gruppe C₁-C₆-Alkyl, Cyclopentyl, Cyclohexyl, Furyl, und unsubstituiertes oder mit ein bis drei F, Cl, C₁-C₄-Alkyl, C₁-C₄-Alkoxy und/oder C₁-C₄-Fluoralkyl substituiertes Phenyl.

Wenn R₁ und R₂ in der Gruppe -PR₁R₂ verschieden sind, dann liegen Liganden vor, die zusätzlich P-chiral sind.

Bei der sekundären Phosphingruppe kann es sich um zyklisches Sekundärphosphino handeln, zum Beispiel solche der Formeln 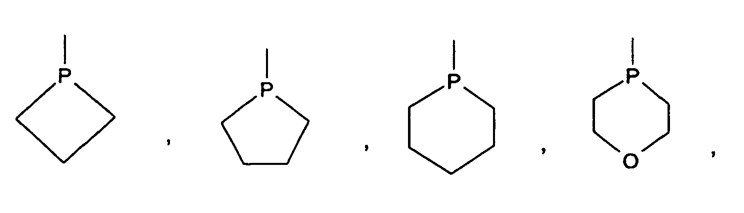 die unsubstituiert oder ein- oder mehrfach, beispielsweise 1-, 2-, 3-, 4-, 5- oder 6-fach, substituiert sind mit Substituenten unabhängig voneinander ausgewählt aus der Gruppe bestehend aus C₁-C₈-Alkyl, C₄-C₈-Cycloalkyl, C₁-C₆-Alkoxy, C₁-C₄-Alkoxy-C₁-C₄-Alkyl, Phenyl, C₁-C₄-Alkyl- oder C₁-C₄-Alkoxyphenyl, Benzyl, C₁-C₄-Alkyl- oder C₁-C₄-Alkoxybenzyl, Benzyloxy, C₁-C₄-Alkyl- oder C₁-C₄-Alkoxybenzyloxy, oder C₁-C₄-Alkyliden-dioxyl.

Die Substituenten können in einer oder beiden α-Stellungen zum P-Atom gebunden sein, um chirale C-Atome einzuführen. Bei den Substituenten in einer oder beiden α-Stellungen handelt es sich bevorzugt um C₁-C₄-Alkyl oder Benzyl, zum Beispiel um Methyl, Ethyl, n- oder iso-Propyl, Benzyl oder -CH₂-O-C₁-C₄-Alkyl beziehungsweise - CH₂-O-C₆-C₁₀-Aryl.

Bei Substituenten in den β,γ-Stellungen kann es sich zum Beispiel um C₁-C₄-Alkyl, C₁-C₄-Alkoxy, Benzyloxy, oder -O-CH₂-O-, -O-CH(C₁-C₄-Alkyl)-O-, und -O-C(C₁-C₄-Alkyl)₂-O- handeln. Einige Beispiele sind Methyl, Ethyl, Methoxy, Ethoxy, -O-CH(Methyl)-O-, und -O-C(Methyl)₂-O-.

An zwei benachbarte C-Atome in den Resten der obigen Formeln kann ein aliphatischer 5-oder 6-Ring oder Benzol ankondensiert sein. Je nach Art der Substitution und Anzahl der Substituenten können die zyklischen Phosphinreste C-chiral, P-chiral oder C- und P-chiral sein.

Das zyklische Sekundärphosphino kann zum Beispiel den nachfolgenden Formeln (nur eines der möglichen Diastereomeren ist angegeben) entsprechen, 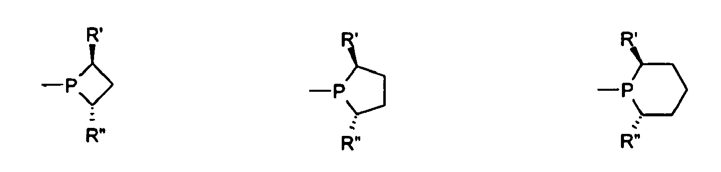 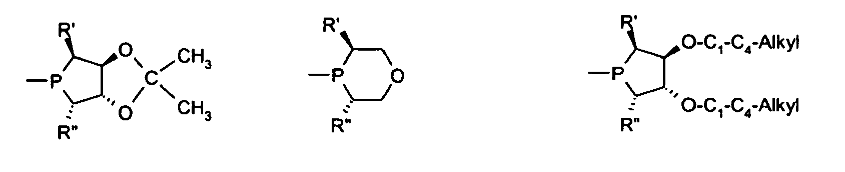 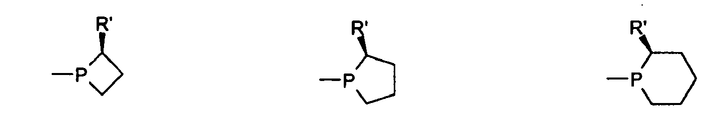 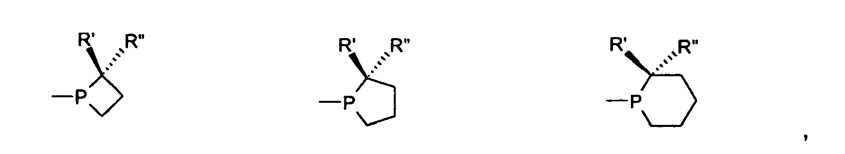 worin
die Reste R' und R" für C₁-C₄-Alkyl, zum Beispiel Methyl, Ethyl, n- oder i-Propyl, Benzyl, oder -CH₂-O-C₁-C₄-Alkyl beziehungsweise -CH₂-O-C₆-C₁₀-Aryl stehen, und R' und R" gleich oder voneinander verschieden sind.

Die zwei Sekundärphosphinoreste -PR₁R₂ in Diphosphinen bedeuten bevorzugt unabhängig voneinander nicht-zyklisches Sekundärphosphin ausgewählt aus der Gruppe -P(C₁-C₆-Alkyl)₂, -P(C₅-C₈-Cycloalkyl)₂, -P(C₇-C₈-Bicycloalkyl)₂, -P(o-Furyl)₂, - P(C₆H₅)₂, -P[2-(C₁-C₆-Alkyl)C₆H₄]2, -P[3-(C₁-C₆-Alkyl)C₆H₄]₂, -P[4-(C₁-C₆-Alkyl)C₆H₄]₂, - P[2-(C₁-C₆-Alkoxy)C₆H₄]₂, -P[3-(C₁-C₆-Alkoxy)C₆H₄]₂, -P[4-(C₁-C₆-Alkoxy)C₆H₄]₂, -P[2-(Trifluormethyl)C₆H₄]₂, -P[3-(Trifluormethyl)C₆H₄]₂, -P[4-(Trifluormethy))C₆H₄]₂, -P[3,5-Bis(trifluormethyl)C₆H₃]₂, -P[3,5-Bis(C₁-C₆-Alkyl)₂C₆H₃]₂, -P[3,5-Bis(C₁-C₆-Alkoxy)₂C₆H₃]₂, und -P[3,5-Bis(C₁-C₆-Alkyl)₂-4-(C₁-C₆-Alkoxy)C₆H₂]₂, oder zyklisches Phosphin, ausgewählt aus der Gruppe

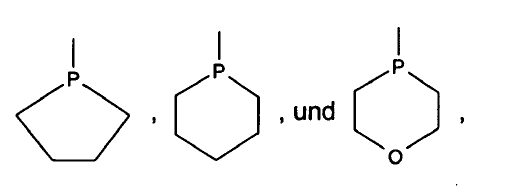

die unsubstituiert oder ein- oder mehrfach, beispielsweise 1-, 2-, 3-, 4-, 5- oder 6fach, substituiert sind mit Substituenten unabhängig voneinander ausgewählt aus der Gruppe bestehend aus C₁-C₄-Alkyl, C₁-C₄-Alkoxy, C₁-C₄-Alkoxy-C₁-C₂-Alkyl, Phenyl, Benzyl, Benzyloxy, und C₁-C₄-Alkyliden-dioxyl.

Einige spezifische Beispiele sind -P(CH₃)₂, -P(iso-C₃H₇)₂, -P(n-C₄H₉)₂, -P(iso-C₄H₉)₂, -P(tert-C₄H₉)₂, -P(C₅H₉), -P(C₆H₁₁)₂, -P(Norbornyl)₂, -P(o-Furyl)₂, -P(C₆H₅)₂, P[2-(Methyl)C₆H₄]₂, P[3-(Methyl)C₆H₄]₂, -P[4-(Methyl)C₆H₄]₂, -P[2-(Methoxy)C₆H₄]₂, -P[3-(Methoxy)C₆H₄]₂, -P[4-(Methoxy)C₆H₄]₂, -P[3-(Trifluormethyl)C₆H₄]₂, -P[4-(Trifluormethyl)C6H₄]_{2,} -P[3,5-Bis(trifiuormethy))C₆H₃]₂, -P[3,5-B is(methyl)C₆H₃]₂, - P[3,5-Bis(methoxy)₂C₆H₃]₂, -P[3,4,5-TrimethoxyC₆H₂]₂, und -P[3,5-Bis(methyl)-4-(methoxy)C₆H₂]₂, und solche der Formeln 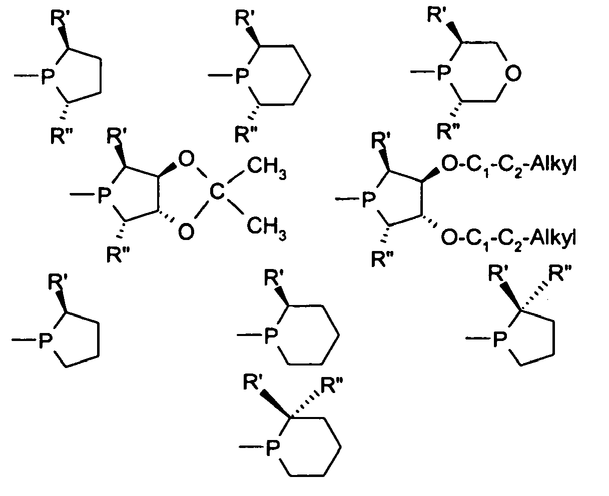 worin
R' Methyl, Ethyl, Methoxy, Ethoxy, Phenoxy, Benzyloxy, Methoxy-methyl, Ethoxymethyl oder Benzyloxymethyl darstellt und R" unabhängig die gleiche Bedeutung wie R' hat.

Bevorzugte Diphosphinliganden sind durch nachfolgende Formeln VII bis XV dargestellt: 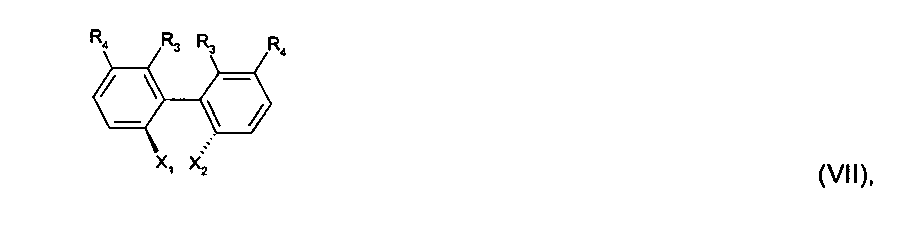 worin X₁ und X₂ für gleiches oder verschiedenes Sekundärphosphino steht, R₃ C₁-C₄-Alkoxy oder C₁-C₄-Alkyl bedeutet, R₄ Wasserstoff ist oder die gleiche Bedeutung wie R₃ hat, oder R₃ und R₄ zusammen mit den C-Atomen, an die sie gebunden sind, einen fünf- oder sechsgliedrigen carbozyklischen Ring oder einen heterozyklischen Ring mit einem oder mehreren, beispielsweise 1 bis 4, Heteroatomen unabhängig voneinander ausgewählt aus der Gruppe O, S, -N= und -N(C₁-C₄-Alkyl) bilden, zum Beispiel können hierzu R₃ und R₄ für einen Rest stehen ausgewählt aus der Gruppe bestehend aus -CH=CH-CH=CH-, -CH=N-CH=CH-, -(CH₂)₃-, -(CH₂)₄-, -CH₂N(C₁-C₄-Alkyl)CH₂-, -N(C₁-C₄-Alkyl)CH₂CH₂-N(C₁-C₄-Alkyl)-, -O-CH₂CH₂-N(C₁-C₄-Alkyl)-, -O-CH₂CH₂-O-, -O-CF₂-O-, -O-CH₂-O-, -O-CH(C₁-C₄-Alkyl)-O-, und -O-C(C₁-C₄-Alkyl)₂-O-; 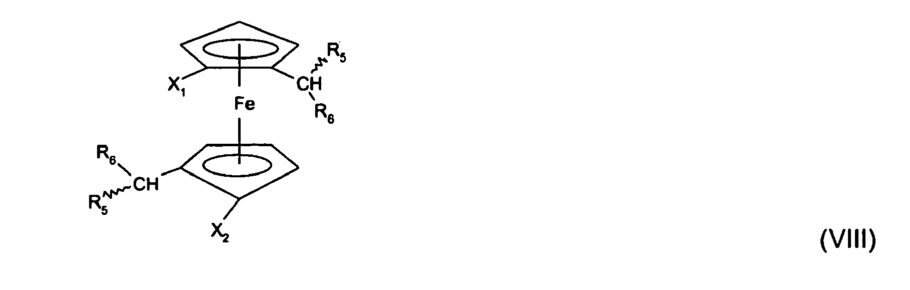 worin X₁ und X₂ für gleiches oder verschiedenes Sekundärphosphino steht, R₆ Sekundäramino, zum Beispiel -N(C₁-C₄-Alkyl)₂ und besonders Dimethylamino bedeutet, und R₅ (C₁-C₆-Alkyl), Cyclohexyl, Phenyl oder Benzyl darstellt; 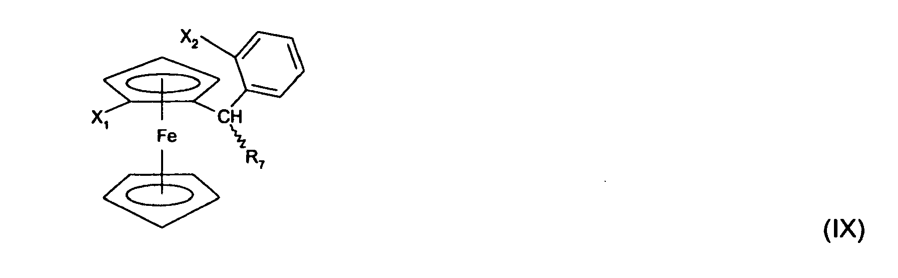 worin X₁ und X₂ für gleiches oder verschiedenes Sekundärphosphino steht und R₇-OH, C₁-C₁₀-Alkoxy, Phenoxy, Benzyloxy oder C₁-C₈-Acyloxy bedeutet; 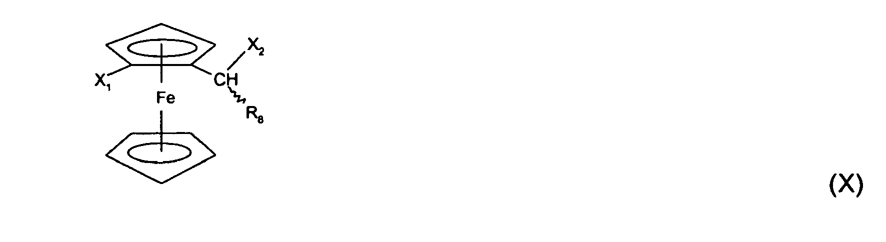 worin X₁ und X₂ für gleiches oder verschiedenes Sekundärphosphino steht und R₈ C₁-C₆-Alkyl, Cyclohexyl, Phenyl oder Benzyl darstellt; 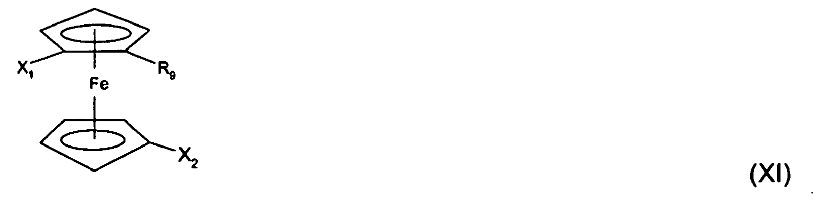 worin X₁ und X₂ für gleiches oder verschiedenes Sekundärphosphino steht und R₉ Wasserstoff bedeutet, oder eine Gruppe -CH(R₇)R₈ darstellt, worin R₇ -OH, C₁-C₁₀-Alkoxy, Phenoxy, Benzyloxy oder C₁-C₈-Acyloxy ist; und R₈ C₁-C₆-Alkyl, Cyclohexyl, Phenyl oder Benzyl bedeutet; 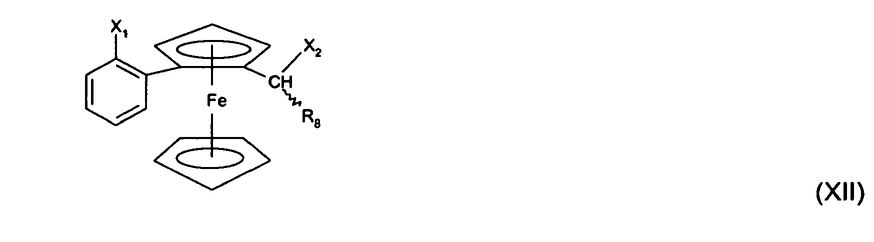 worin X₁ und X₂ für gleiches oder verschiedenes Sekundärphosphino steht und R₈ C₁-C₆-Alkyl, Cyclohexyl, Phenyl oder Benzyl bedeutet; 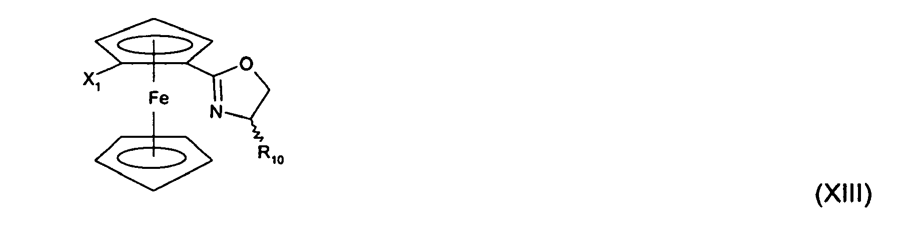 worin X₁ für Sekundärphosphino steht und R₁₀ C₁-C₆-Alkyl, Cyclohexyl, Phenyl oder Benzyl bedeutet; 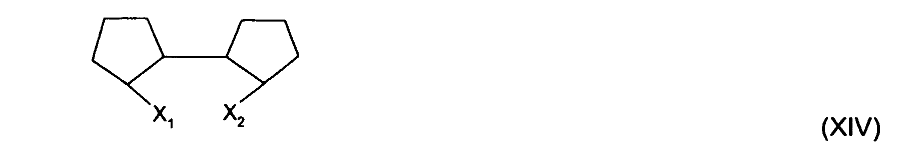 worin X₁ und X₂ für gleiches oder verschiedenes Sekundärphosphino steht; und P-chirale Ethylen-1,2-diphosphine der Formel XV 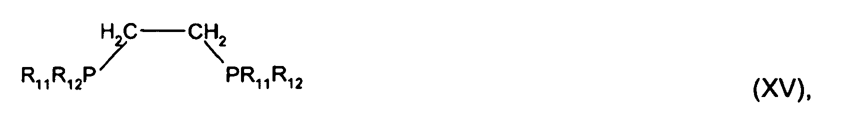 worin R₁₁ und R₁₂ verschiedene Kohlenwasserstoffreste mit 1 bis 20 C-Atomen darstellen, die unsubstituiert oder mit C₁-C₆-Alkyl und/oder C₁-C₆-Alkoxy substituiert sind. Ein bekannter Vertreter ist "dipamp", wenn R₁₁ Phenyl und R₁₂ α-Methoxyphenyl sind.

Diphosphinliganden, besonders 1,1'-Binaphthyl-6,6'-disekundärphosphine, können in Rutheniumkomplexen zusammen mit chiralen Diaminen verwendet werden (Noyori Technik), zum Beispiel 1,2-Diphenyl-ethylen-1,2-diamin (dpen) und 1,1-Dibenzyl-2-isopropyl-1,2-diamin (daipen).

Die Metallkomplexe der Rh- Ir- Ru- Katalysatoren mit chiralem Diphosphinliganden können je nach Oxidationszahl und Koordinationszahl des Metalls weitere Liganden und/oder Anionen enthalten. Es kann sich auch um kationische Metallkomplexe handeln. Solche analogen Metallkomplexe und deren Herstellung sind vielfach in der Literatur beschrieben.

Die Metallkomplexe können zum Beispiel den allgemeinen Formeln XVI und XVII entsprechen,

A₁MeLₙ (XVI),

(A,MeLₙ)^{(z+)}(E⁻)_{z} (XVII),

worin A₁ für einen Diphosphin-Liganden, einschliesslich der Ausgestaltungen und Bevorzugungen, insbesondere der Formeln VII bis XV, steht,
L für gleiche oder verschiedene monodentate, anionische oder nicht-ionische Liganden steht, oder zwei L für gleiche oder verschiedene bidentate, anionische oder nicht-ionische Liganden steht;
n für 2, 3 oder 4 steht, wenn L einen monodentaten Liganden bedeutet, oder n für 1 oder 2 steht, wenn L einen bidentaten Liganden bedeutet;
z für 1, 2 oder 3 steht;
Me = Rhodium (Rh), Iridium (Ir) und Ruthenium (Ru), bevorzugt Rh und Ir bedeutet; wobei das Metall die Oxidationsstufen 0, 1, 2, 3 oder 4 aufweist;
E⁻ das Anion einer Sauerstoffsäure oder Komplexsäure ist; und
die anionischen Liganden die Ladung der Oxidationsstufen 1, 2, 3 oder 4 des Metalls ausgleichen.

Monodentate nicht-ionische Liganden können zum Beispiel ausgewählt sein aus der Gruppe der Olefine (zum Beispiel Ethylen, Propylen), Allyle (z.B. Allyl, 2-Methallyl), solvatisierenden Lösungsmitteln (z.B. Nitrile, lineare oder cyclische Ether, gegebenenfalls N-alkylierte Amide und Lactame, Amine, Phosphine (besonders tertiäre Phosphine wie Triphenylphosphin), Alkohole, Carbonsäureester, Sulfonsäurester, Stickstoffmonoxid, Kohlenmonoxid und Arene (z.B. Benzol, Mesitylen, Cumol).

Monodentate anionische Liganden können zum Beispiel ausgewählt sein aus der Gruppe Cyclopentadienyl, Hydrid, Halogenid (z.B. F, Cl, Br, I), Pseudohalogenid (z.B. Cyanid, Cyanat, Isocyanat) und Anionen von Carbonsäuren, Sulfonsäuren und Phosphonsäuren (z.B. Carbonat, Formiat, Acetat, Propionat, Methylsulfonat, Trifluormethylsulfonat, Phenylsulfonat, Tosylat).

Bidentate nicht-ionische Liganden können zum Beispiel ausgewählt sein aus der Gruppe der linearen oder cyclischen Diolefine (zum Beispiel Hexadien, Cyclooctadien, Norbomadien), Dinitrile (z.B. Malondinitril), gegebenenfalls N-alkylierte Carbonsäurediamide, Diaminen, Diphosphinen, Diolen, Acetonylacetonate, Dicarbonsäurediester und Disulfonsäurediester.

Bidentate anionische Liganden können zum Beispiel ausgewählt sein aus der Gruppe der Anionen von Dicarbonsäuren, Disulfonsäuren und Diphosphonsäuren (zum Beispiel von Oxalsäure, Malonsäure, Bernsteinsäure, Maleinsäure, Methylendisulfonsäure und Methylendiphosphonsäure).

Bevorzugte Metallkomplexe sind auch solche, worin E für -Cl⁻, -Br⁻, -I⁻, ClO₄⁻, CF₃SO₃⁻, CH₃SO₃⁻, HSO₄⁻, BF₄⁻, B(Phenyl)₄⁻, B(C₆F₅)₄⁻, B(3,5-Bistrifluormethyl-phenyl)₄⁻, PF₆⁻, SbCl₆⁻, AsF₆⁻ oder SbF₆⁻ steht.

Bevorzugte Rh- und Ir-Metallkomplexe entsprechen den Formeln XVIII und IXX,

[A₁Me₁YZ] (XVIII),

[A₁Me₁Y]⁺E₁⁻ (IXX),

worin
A₁ für einen Diphosphin-Liganden, einschliesslich der Ausgestaltungen und Bevorzugungen, insbesondere der Formeln VII bis XV, steht;
Me₁ Rhodium (Rh) und Iridium (Ir) bedeutet;
Y für zwei Olefine oder ein Dien steht;
Z Cl, Br, I oder BF₄- bedeutet; und
E₁⁻ das Anion einer Sauerstoffsäure oder Komplexsäure darstellt.

Bei Y in der Bedeutung als Olefin kann es sich um C₂-C₁₂-, bevorzugt C₂-C₆- und besonders bevorzugt C₂-C₄-Olefine handeln. Beispiele sind Propen, But-1-en und besonders Ethylen. Das Dien kann 5 bis 12 und bevorzugt 5 bis 8 C-Atome enthalten und es kann sich um of-fenkettige, cyclische oder polycyclische Diene handeln. Die beiden Olefingruppen des Diens sind bevorzugt durch ein oder zwei CH₂-Gruppen verbunden. Beispiele sind 1,3-Pentadien, Cyclopentadien, 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien und Norbornadien.

Bevorzugt stellt Y zwei Ethylen oder 1,5-Hexadien, 1,5-Cyclooctadien oder Norbornadien dar.

In Formel XVIII steht Z bevorzugt für Cl oder Br. Beispiele für E₁ sind ClO₄⁻, CF₃SO₃⁻, CH₃SO₃⁻, HSO₄⁻, BF₄⁻, B(Phenyl)₄⁻, PF₆⁻, SbCl₆⁻, AsF₆⁻ oder SbF₆⁻. Rutheniumkomplexe können zum Beispiel der Formel XX entsprechen,

[RuₐH_{b}Z_{c}(A₁)_{d}Lₑ]_{f}(E^{k})_{g}(S)ₕ (XX),

worin
Z Cl, Br oder I bedeutet; A₁ für einen Diphosphinliganden und bevorzugt für ein Diphosphin der Formeln VII bis XV steht; L für gleiche oder verschiedene Liganden steht; E^{k-} das Anion einer Sauerstoffsäure, Mineralsäure oder Komplexsäure ist; S für ein koordinationsfähiges Lösungsmittel als Ligand steht; und a 1 bis 3, b 0 bis 4, c 0 bis 6, d 1 bis 3, e 0 bis 4, f 1 bis 3, g 1 bis 4, h 0 bis 6 und k 1 bis 4 bedeuten, wobei die Gesamtladung des Komplexes neutral ist.

Für die Verbindungen der Formel XX gelten die zuvor dargestellten Bevorzugungen für Z, A₁, L und E⁻. Bei den Liganden L kann es sich zusätzlich um Arene oder Heteroarene (zum Beispiel Benzol, Naphthalin, Methylbenzol, Xylol, Cumol, 1,3,5-Mesitylen, Pyridin, Biphenyl, Pyrrol, Benzimidazol oder Cyclopentadienyl) und Metallsalze mit Lewissäurefunktion (zum Beispiel ZnCl₂, AlCl₃, TiCl₄ und SnCl₄) handeln. Bei den Lösungsmittelliganden kann es sich zum Beispiel um Alkohole, Amine, Säureamide, Lactame und Sulfone handeln.

Komplexe dieser Art sind in der nachfolgend erwähnten Literatur und der darin zitierten Literatur beschrieben:
D. J. Ager, S. A. Laneman, Tetrahedron: Asymmetry, 8, 1997, 3327 - 3355;
T. Ohkuma, R. Noyori in Comprehensive Asymmetric Catalysis (E.N. Jacobsen, A. Pfaltz, H. Yamamoto, Eds.), Springer, Berlin, 1999, 199-246;
J. M. Brown in Comprehensive Asymmetric Catalysis (E.N. Jacobsen, A. Pfaltz, H. Yamamoto, Eds.), Springer, Berlin, 1999, 122 - 182;
T. Ohkuma, M. Kitamura, R. Noyori in Catalytic Asymmetric Synthesis, 2nd Edition (I. Ojima, Ed.), Wiley-VCH New York, 2000, 1 - 110;
N. Zanetti, et al. Organometallics 15, 1996, 860.

Die Metallkomplexe werden nach in der Literatur bekannten Methoden hergestellt (siehe z.B. Comprehensive Asymmetric Catalysis I bis III, Springer Verlag, Berlin, 1999, und darin zitierte Literatur).

In einer besonders bevorzugten Ausführungsform des erfindungsgemäßen Verfahrens werden Rutheniumkomplexe als homogene und chirale Hydrierkatalysatoren verwendet, wobei mit solchen des Typs [RuHalogenid₂(diphosphin Formel VII)(chirales Diamin)] und des Typs [RuHalogenid₂(Ligand Formel XIII)(tertiäres Phosphin)] hinsichtlich Selektivität, Reaktionszeit (Aktivität), vollständigem Umsatz und Bildung nur sehr geringer Mengen Nebenprodukt besonders herausragende Ergebnisse erzielt werden. Halogenid ist bevorzugt Cl, Br und I.

Es kann vorteilhaft sein, die homogene Hydrierung in Gegenwart von Zusätzen durchzuführen. Die Menge kann bis zu äquimolaren Mengen oder mehr reichen, bezogen auf die Verbindungen der Formeln I und II. Einige Beispiele sind Säuren, anorganische Basen (zum Beispiel NaOH) und Alkalimetallalkoholate (zum Beispiel Kalium-t-butylat).

Besonders zweckmässig kann es sein, zum Beispiel wenn das Substrat als freie Base eingesetzt wird, die Hydrierung in Gegenwart von Säuren durchzuführen, zum Beispiel organischen Säuren wie Sulfonsäuren (z.B. Methansulfonsäure, Trifluormethansäure), Carbonsäuren (z.B. Ameisensäure, Essigsäure, Oxalsäure), Phosphonsäuren (z.B. Methanphosphonsäure), Mineralsäuren wie Halogenwasserstoffsäuren (z.B. HCl, HBr, Hl), Schwefelsäure, phosphorige Säure, Phosphorsäure (siehe zum Beispiel US-A-5,371,256, US-A-5,446,844 und US-A-5,583,241 und EP-A-0 691 949). Die Säure kann so gewählt werden, dass man direkt ein gewünschtes Salz des Wirkstoffs erhält. Entsprechend kann die Menge an Säure bis zu 1 Äquivalent oder mehr betragen, zum Beispiel ein Überschuss bis zu 1,5 Äquivalente, bezogen auf die Menge des zu hydrierenden Substrats. Ein geeigneter Mengenbereich ist 0,01 bis 1 Äquivalente Säure, bezogen auf die Menge des zu hydrierenden Substrats.

Die als Katalysatoren verwendeten Metallkomplexe können als getrennt hergestellte isolierte Verbindungen zugegeben werden, oder bevorzugt auch in situ vor der Reaktion gebildet und dann mit dem zu hydrierenden Substrat vermischt werden. Es kann vorteilhaft sein, bei der Reaktion unter Verwendung von isolierten Metallkomplexen zusätzlich Liganden zuzugeben, oder bei der in situ Herstellung einen Überschuss der Liganden einzusetzen. Der Überschuss kann zum Beispiel 1 bis 10 und vorzugsweise 1 bis 5 Molprozent betragen, bezogen auf die zur Herstellung verwendete Metallverbindung.

Das erfindungsgemäße Verfahren wird im Allgemeinen so durchgeführt, dass man den Katalysator vorlegt und dann das Substrat, gegebenenfalls Reaktionshilfsmittel und die gasförmige anzulagernde Verbindung in Form von Wasserstoff vorzugsweise aufpresst. Das Verfahren kann in verschiedenen Reaktortypen kontinuierlich oder satzweise durchgeführt werden.

Erfindungsgemäß zum Einsatz kommende Alkalimetall-trialkylborhydride und Alkalimetall-trialkylaluminiumhydride können insbesondere den Formeln XXI und XXIa entsprechen,

Alkalimetall[B(R₁₃)₃H] (XXI),

Alkalimetall[Al(R₁₃)₃H] (XXIa),

worin Alkalimetall für Li, Na oder K steht und R₁₃ ein linearer oder verzweigter Alkylrest mit 1 bis 18, bevorzugt 3 bis 18, bevorzugter 4 bis 12 und besonders bevorzugt 4 bis 8 C-Atomen ist. Wenn R₁₃ verzweigtes Alkyl ist, kann es wenigstens ein asymmetrisches C-Atom aufweisen, was aber nicht so entscheidend ist wie der Einfluss der räumlichen Beanspruchung auf die Selektivität.

R₁₃ ist zum Beispiel bevorzugt in α-Stellung verzweigt. R₁₃ kann aber auch in den β-, γ- und/oder δ-Stellungen verzweigt sein. Beispiele sind But-2-yl, Pent-2-yl, Hex-2-yl, Hept-2-yl, Oct-2-yl, 2- und 1,2-Dimethyl-but-1-yl, 2-Ethyl-pent-1-yl, Hex-2-yl, 2-Methyl- oder 2-Ethyl-hex-1-yl, 1,2,2-Trimethyl-eth-1-yl und 1,2-Dimethyl-but-1-yl.

Verbindungen der Formeln XXI und XXIa sind teilweise käuflich erhältlich, zum Beispiel unter der Bezeichnung Selectride^{®}, oder sie können nach bekannten Verfahren in analoger Weise hergestellt werden. Die Stereoselektivität ist besonders hoch und das Verhältnis von Diastereomeren der Formeln III und IV zu Diastereomeren der Formeln V und VI kann >99,5:<0,5 betragen. Die Reaktivität ist hoch und es können bei kurzen Reaktionszeiten vollständige Umsätze erzielt werden. Die Bildung von Nebenprodukten kann besonders bei Temperaturen von - 20°C und darunter wirksam unterdrückt werden auf Mengen von etwa 1 bis weniger als 10 Gew.-%, bezogen auf das Reaktionsprodukt. Es kann zweckmässig sein, den Reaktionsverlauf zu beobachten und das Reaktionsende zu bestimmen, um unnötig lange Reaktionszeiten zu vermeiden, da hierbei eine Zersetzung des Produktes eintreten kann. Als Lösungsmittel (Reaktionsmedien) eignen sich besonders Ether wie zum Beispiel Diethylether, Diisopropylether, Dibutylether, Ethylenglykoldimethylether, Dioxan und Tetrahydrofuran.

Die nachfolgenden Beispiele erläutern die Erfindung näher ohne sie hierauf zu beschränken.

### Beispiele:

Das Ausgangsprodukt in Form des Racemats von (3R,4R)-4-Dimethylaminomethyl-3-hydroxy-3-(3-methoxyphenyl)-cyclohexanon und (3S,4S)-4-Dimethylaminomethyl-3-hydroxy-3-(3-methoxyphenyl)-cyclohexanon (Verbindungen der Formeln I und II, in denen jeweils R für CH₃ steht) wird nachfolgend als cis-1 bezeichnet. Die Herstellung ist beispielsweise in EP-A1-0 753 506 (Beispiel 18) beschrieben. Cis-1 kann mittels bekannter Trennmethoden wie beispielsweise fraktionierter Kristallisation oder chromatographischer Trennung von den anderen Stereoisomeren (3R,4S)-4-Dimethylaminomethyl-3-hydroxy-3-(3-methoxyphenyl)-cyclohexanon und (3S,4R)-4-Dimethylaminomethyl-3-hydroxy-3-(3-methoxyphenyl)-cyclohexanon getrennt werden.

Das gemäß dem jeweiligen Beispiel erhaltene Reaktionsgemisch wurde mittels Hochdruckflüssigkeitschromatographie (HPLC) mit einer Säule Nucleosil C8, 25 x 4,6 mm, 5 µm bei Raumtemperatur untersucht. Es wurden zwei mobile Phasen verwendet: A) Acetonitril / Wasser / 0,5M KH₂PO₄ aq. = 5 / 90 /5; und B) Acetonitril /Wasser / 0,5M KH₂PO₄ aq. = 60 / 35 / 5, und zwar mit folgendem Gradienten (Volumenprozente): 0 Minuten / 90%A / 10%B; 50 Minuten / 100% B; 55 Minuten 100% B; 56 Minuten 90% A / 10% B. Der Fluss betrug 20 µl und die Detektion wurde bei 220 nm vorgenommen. Die Produkte wurden in folgender Reihenfolge eluiert: Nach 12,5 Minuten Diastereomere der Formeln V und VI, nach 14,9 Minuten die Verbindungen der Formeln I und II (cis-1) und nach 18,5 Minuten Diastereomere der Formeln III und IV (gewünschtes Produkt).

Das Verhältnis von Diastereomeren der Formeln III und IV zu Diastereomeren der Formeln V und VI wird als Selektivität bezeichnet. Die nachstehend angegebenen Gewichtsprozente der Katalysatoren beziehen sich jeweils auf die eingesetzte Menge an cis-1.

### A) Heterogene Hydrierung

### Beispiel A1: Hydrierung mit PtO₂

500 mg (1,8 mmol) cis-1 und 5 ml Ethanol wurden zusammen mit 1,1 Äquivalenten Eisessig und 15 mg PtO₂ (3 Gew.-%) in einen Glaskolben gegeben und der Glaskolben verschlossen. Dann wurde der Kolben in vier Zyklen mit Argon und in 4 Zyklen mit Wasserstoff gespült. Es wurden 1,1x10⁵ Pa Wasserstoff aufgepresst und die Reaktion durch Anstellen des Rührers gestartet. Es wurde 24 Stunden bei Raumtemperatur gerührt, der Glaskolben mit Argon gespült und anschließend der Katalysator abfiltriert. Das Filtrat wurde chromatographisch untersucht. Der Umsatz betrug 91% und die Selektivität 93:7.

Bei einer Erhöhung der Katalysatorkonzentration auf 10 Gew.-% wurde unter sonst gleichen Bedingungen ein vollständiger Umsatz bei einer Selektivität von 95:5 erzielt.

### Beispiel A2: Hydrierung mit Raney-Nickel

200 mg (0,72 mmol) cis-1, 2 ml Lösungsmittel und mit Wasser angefeuchtetes Raney-Nickel (RaNi; Typ H467, Firma Engelhard, jetzt BASF) wurden in einen Glaskolben gegeben, der Glaskolben in einen Autoklaven gesetzt und der Autoklav verschlossen. Dann wurde der Autoklav in vier Zyklen mit Argon und in 4 Zyklen mit Wasserstoff gespült. Es wurde Wasserstoff aufgepresst und die Reaktion durch Anstellen des Rührers gestartet. Es wurde zumindest bis zum Reaktionsende gerührt, der Glaskolben mit Argon gespült und anschliessend der Katalysator abfiltriert. Das Filtrat wurde chromatographisch untersucht. Die detaillierten Angaben zur Umsetzung sowie die Ergebnisse finden sich in nachfolgender Tabelle 1.

Die Menge an RaNi ist in Gewichtsprozent (w/w-%) angegeben (berechnet als wasserfeuchter Katalysator, Wassergehalt ca. 50 Gew.-%). Die Temperatur (T) - sofern nicht Raumtemperatur (RT) - ist in °C angegeben. Der Wasserstoffdruck ist in 10⁵ Pa angegeben. Die Reaktionszeit ist in Stunden angegeben. Die Prozentangaben für cis-1, Diastereomere der Formeln III und IV (III/IV), Diastereomere der Formeln V und VI (V/VI) und SAN (Summe aller Nebenprodukte) sind chromatographisch bestimmte Flächenprozente. MeOH ist Methanol, EtOH ist Ethanol. EtAc ist Ethylacetat. iPrOH ist Isopropanol. Tol ist Toluol.

**Tabelle 1:**

| Menge RaNi | Lösungsmittel | T | Druck | Zeit | cis-1 (%) | III/IV (%) | V/VI (%) | SAN (%) | Selektivität |
|---|---|---|---|---|---|---|---|---|---|
| 40 | MeOH | RT | 5 | 17,3 | 0 | 96 | 4 | 0 | 96:4 |
| 40 | MeOH | RT | 50 | 40,3 | 0 | 95 | 4 | 1 | 96:4 |
| 40 | EtOH | RT | 6 | 24 | 0 | 95 | 3 | 2 | 97:3 |
| 20 | EtOH | RT | 6 | 24 | 14 | 78 | 3 | 5 | 96:4 |
| 10 | EtOH | RT | 6 | 24 | 37 | 55 | 2 | 6 | 96:4 |
| 5 | EtOH | RT | 6 | 24 | 55 | 36 | 1 | 8 | 97:3 |
| 40 | EtAc | RT | 6 | 24 | 3 | 87 | 9 | 1 | 91:9 |
| 20 | iPrOH | RT | 6 | 24 | 13 | 80 | 3 | 4 | 96:4 |
| 20 | Tol | RT | 6 | 24 | 4 | 90 | 4 | 2 | 96:4 |
| 20 | Tol | RT | 10 | 6,6 | 55 | 39 | 2 | 4 | 95:5 |
| 20 | Tol | RT | 10 | 23,3 | 29 | 62 | 3 | 7 | 95:5 |
| 40 | Tol | RT | 5 | 4,8 | 66 | 25 | 1 | 8 | 96:4 |
| 40 | Tol | RT | 5 | 27,2 | 2 | 85 | 3 | 10 | 97:3 |
| 20^{a)} | Tol | RT | 20 | 24 | 76 | 8 | 0 | 16 | 100:0 |
| 20^{a)} | Tol | 60 | 20 | 24 | 8 | 79 | 3 | 10 | 96:4 |
| 20^{a)} | Tol | 0 | 20 | 24 | 79 | 8 | 0 | 13 | 100:0 |
| 20^{a)} | Tol | 60 | 5 | 24 | 64 | 16 | 1 | 19 | 94:6 |
| 40^{b)} | Tol | RT | 5 | 24 | 36 | 61 | 2 | 1 | 97:3 |
| 40^{b)} | Tol | RT | 5 | 24 | 37 | 60 | 2 | 1 | 97:3 |
| 40^{b,c)} | EtOH | RT | 5 | 24 | 3 | 92 | 2 | 3 | 98:2 |

| | | | | | | | | | |
|---|---|---|---|---|---|---|---|---|---|
| ^{a)} cis-1 enthielt bereits Zersetzungsprodukte (Braunfärbung) ^{b)} cis-1 wurde vor der Hydrierung aus Diisopropylether umkristallisiert ^{c)} Variante von Beispiel A2: 50 ml Stahlautoklav, 2g (7,2 mmol) cis-1 und 20 ml Lösungsmittel | | | | | | | | | |

### Beispiel A3: Hydrierung mit Raney-Nickel

Das Verfahren von Beispiel A2 wurde mit 40 Gew.-% RaNi (Wassergehalt ca. 50 Gew.-%) wiederholt und hierbei die Reaktionsparameter variiert. Es zeigte sich, dass eine höhere Temperatur die Selektivität geringfügig erniedrigt und die Katalysatoraktivität nur wenig beeinflusst. Mit einem höheren Druck können kürzere Reaktionszeiten erzielt werden, wobei sich die Selektivität praktisch nicht verändert. Die Zugabe von Additiven (zum Beispiel 0,5 Äquivalente MgCl₂, 1,5 Äquivalente Eisessig oder 50 Gew.-% NaOH, jeweils bezogen auf eingesetztes cis-1) führt zu einer starken Erhöhung von Nebenprodukten im Reaktionsgemisch. Einzelheiten sind in Tabelle 2 wiedergegeben.

**Tabelle 2:**

| T | Druck | Zeit | cis-1 | III/IV | V/VI | SAN | Selektivität |
|---|---|---|---|---|---|---|---|
| | | | (%) | (%) | (%) | (%) | |
| RT | 5 | 24 | 3 | 92 | 2 | 3 | 98:2 |
| 60 | 5 | 28 | 0 | 91 | 5 | 4 | 95:5 |
| 40 | 5 | 27 | 0 | 94 | 4 | 2 | 96:4 |
| 40 | 10 | 24 | 0 | 90 | 6 | 4 | 94:6 |
| 40 | 20 | 24 | 0 | 89 | 3 | 8 | 96:4 |
| 40 | 1,1 | 24 | 17 | 70 | 3 | 10 | 96:4 |
| 40^{a)} | 5 | 24 | 7 | 64 | 3 | 26 | 96:4 |
| 40 | 5 | 24 | 0 | 89 | 3 | 8 | 96:4 |
| 40^{b)} | 5 | 24 | 0 | 9 | <1 | 90 | 98:2 |
| 40^{c)} | 5 | 21 | 0 | 27 | 0 | 73 | 100:0 |

| | | | | | | | |
|---|---|---|---|---|---|---|---|
| ^{a)} Zusatz von 0,5 Äquivalenten MgCl₂, ^{b)} 1,5 Äquivalente Eisessig beziehungsweise ^{c)} 50 Gew.-% NaOH. In den letzten 4 Versuchen enthält cis-1 bereits Zersetzungsprodukte (Braunfärbung). | | | | | | | |

### Beispiel A4: Hydrierung mit Raney-Nickel

Beispiel A2 wurde mit 40 Gew.-% verschiedenen feuchten Katalysatortypen von den Firmen Engelhard und Degussa in Ethanol bei 40 °C und 5x10⁵ Pa wiederholt. Die Reaktionszeit betrug 24 Stunden. Der Einfluss der Katalysatortypen auf das Ergebnis ist gering. Das Ausgangsprodukt cis-1 enthielt etwa 6 % Nebenprodukte. Einzelheiten sind in nachfolgender Tabelle 3 angegeben.

**Tabelle 3:**

| RaNi Typ | cis-1 | III/IV | V/VI | SAN | Selektivität |
|---|---|---|---|---|---|
| | (%) | (%) | (%) | (%) | |
| EtOH-RaNi H467 | 0 | 89 | 3 | 8 | 96:4 |
| RaNi B111W | 0 | 85 | 6 | 9 | 93:7 |
| RaNi Actimet M | 0 | 88 | 4 | 8 | 95:5 |
| RaNi B113W | 0 | 87 | 7 | 6 | 92:8 |
| RaNi BLM 112 W | 0 | 86 | 6 | 8 | 93:7 |
| RaNi BP 113EXP-B | 0 | 87 | 7 | 6 | 92:8 |
| RaNi Actimet M (mit EtOH gewaschen) | 0 | 91 | 6 | 3 | 94:6 |

Beispiel A5: Hydrierung mit Raney-Nickel und Wiederverwendung des Katalysators 3,0 g (10,8 mmol) cis-1, suspendiert in 80 ml Ethanol wurden in einen 300 ml Stahlautoklaven mit gesinterter Filterplatte gegeben. Dann wurden 1,2 g mit Ethanol angefeuchtetes Raney-Nickel (RaNi; Typ H467, Firma Engelhard, suspendiert in 20 ml Ethanol) zugefügt und der Autoklav verschlossen. Dann wurde der Autoklav in drei Zyklen mit Argon und in drei Zyklen mit Wasserstoff gespült. Es wurde Wasserstoff aufgepresst (4x10⁵ Pa) und die Reaktion durch Anstellen des Rührers gestartet. Es wurde 24 Stunden gerührt, mit Stickstoff gespült, anschliessend der Katalysator abfiltriert und mit Ethanol gewaschen. Das Filtrat wurde chromatographisch untersucht. Bei der 3. Wiederverwendung wurde der Katalysator vor dem erneuten Einsatz mit Ethanol, 0,5N NaOH und mit Methanol gewaschen, das Startmaterial cis-1 enthielt bereits etwa 25% Nebenprodukte. Weitere Angaben und die Ergebnisse finden sich in nachfolgender Tabelle 4.

**Tabelle 4:**

| Anmerkung | cis-1 | III/IV | V/VI | SAN | Selektivität |
|---|---|---|---|---|---|
| | (%) | (%) | (%) | (%) | |
| Startreaktion | 0 | 79 | 5 | 16 | 94:6 |
| 1. Wiederverwendung | 6 | 77 | 5 | 12 | 94:6 |
| 2. Wiederverwendung | 23 | 56 | 2 | 19 | 98:2 |
| 3. Wiederverwendung | 0 | 59 | 1 | 40 | 98:2 |

### Beispiel A6: Hydrierung mit Raney-Nickel und ansteigender Menge an Ausgangsprodukt

Beispiel A2 wurde wiederholt und steigende Mengen cis-1 eingesetzt. Die ersten 4 Versuche wurden gemäß der Variante von Beispiel A2 im 50 ml Stahlautoklav durchgeführt. Das Startmaterial cis-1 im ersten Versuch enthielt bereits etwa 6% Nebenprodukte. Weitere Einzelheiten finden sich in Tabelle 5.

**Tabelle 5:**

| mg cis-1 / ml | T | Druck | Zeit | cis-1 (%) | III/IV (%) | V/VI (%) | SAN (%) | Selektivität |
|---|---|---|---|---|---|---|---|---|
| 100 | 45 | 9 | 23 | 1 | 89 | 3 | 7 | 97:3 |
| 200 | 45 | 9 | 23 | 0 | 94 | 3 | 3 | 97:2 |
| 267 | 45 | 9 | 24 | 0 | 91 | 4 | 5 | 96:4 |
| 333 | 45 | 9 | 24 | 0 | 94 | 4 | 2 | 98:2 |
| 500 | 40 | 5 | 24 | 0 | 95 | 3 | 2 | 97:3 |
| 1000 | 40 | 5 | 24 | 0 | 96 | 3 | 1 | 97:3 |
| 2000 | 40 | 5 | 24 | 26 | 70 | 1 | 3 | 98:2 |

Beispiel A7: Hydrierung mit Raney-Nickel mit grösserer Menge an Ausgangsprodukt Beispiel A5 wurde mit 20 g cis-1 bei 40 °C in Gegenwart von 40 Gew.-% RaNi (H₂O-H467 bzw. EtOH-H467) in Ethanol bei einer Konzentration von 1000 mg cis-1 pro ml Ethanol wiederholt (Versuche 1 und 2). Das Startmaterial cis-1 in den Versuchen 1 und 2 enthielt etwa 2 % Nebenprodukte.
Beispiel A1 wurde mit 50 g cis-1 bei 40 °C in Gegenwart von 40 Gew.-% RaNi (Actimet M) in Ethanol bei einer Konzentration von 1000 mg cis-1 pro ml Ethanol wiederholt (Versuch 3). Das Startmaterial cis-1 in Versuch 3 enthielt etwa 15% Nebenprodukte. Vor der Filtration muss ungelöstes Material durch Zugabe von Lösungsmittel gelöst werden. Die Ergebnisse sind in Tabelle 6 zusammengefasst.

**Tabelle 6:**

| RaNi Typ | Druck | Zeit | cis-1 (%) | III/IV (%) | V/VI (%) | SAN (%) | Selektivität |
|---|---|---|---|---|---|---|---|
| H₂O-H467^{a)} | 9,5 | 23 | 0 | 88 | 8 | 4 | 92:8 |
| EtOH-H467^{a)} | 9,5 | 3 | 0 | 87 | 9 | 4 | 91:9 |
| Actimet M^{b)} | 7 | 2^{c)} | 2 | 73 | 5 | 20 | 93:7 |

| | | | | | | | |
|---|---|---|---|---|---|---|---|
| ^{a)} Firma Engelhard, ^{b)} Firma Degussa, ^{c)} Aufnahme Wasserstoff nach 30 Minuten beendet. | | | | | | | |

### B) Homogene Hydrierung

Den verwendeten Liganden kommen folgende Strukturen zu:
L1: Formel VII, R₃ und R₄ sind zusammen -CH=CH-CH=CH-, X₁ und X₂ sind je - P(C₆H₅)₂.
L2: Formel XIII, R₁₀ ist t-Butyl, X₁ ist -P(C₆H₅)₂.
L3: Formel XIII, R₁₀ ist i-Propyl, X₁ ist -P(C₆H₅)₂.
L4: Formel XIII, R₁₀ ist i-Propyl, X₁ ist -P[(3,5-Dimethyl-4-methoxyC₆H₂)]₂.
L5: Formel XIII, R₁₀ ist Phenyl, X₁ ist -P(C₆H₅)₂.
L6: Formel VII, R₃ und R₄ sind zusammen -O-CH₂CH₂-N(CH₃)-, X₁ und X₂ sind je - P[(3,5-Dimethyl-CsH₃)]₂. L7: Formel VII, R₃ und R₄ sind zusammen -O-CF₂-O-, X₁ und X₂ sind je -P(C₆H₅)₂.
L8: Formel XII, R₈ Methyl, X₁ ist -P(C₆H₅)₂, X₂ ist -P[(3,5-Di(trifluormethyl)-C₆H₃)]₂ (R,R-Konfiguration).
L9: Formel XIV, X₁ und X₂ sind je -P(C₆H₅)₂.
L10: Formel X, R₈ ist Methyl, X₁ ist -P[(3,5-Dimethyl-C₆H₃)]₂, X₂ ist -P[(3,5-Di(trifluormethyl)-C₆H₃)]₂.
L11: Formel VII, R₃ und R₄ sind zusammen -O-CH₂CH₂-N(CH₃)-, X₁ und X₂ sind je - P[(3,5-Dimethyt-4-methoxy-C₆H₂)]₂.
L12: Formel XII, R₈ ist Methyl, X₁ ist -P[(3,5-Dimethyl-4-methyl-C₆H₂)]₂, X₂ ist -P[(3,5-Di(trifluormethyl)-C₆H₃)]₂.
L13: Formel VIII, R₅ ist Phenyl und R₆ ist Dimethylamino, X₁ und X₂ sind je -P[(3,5-Dimethyl-4-methoxy-C₆H₂)]₂.
L14: Formel XII, R₈ Methyl, X₁ und X₂ sind je -P(C₆H₅)₂.
L15: Formel X, R₈ ist Methyl, X₁ ist -P(C₆H₅)₂, X₂ ist -P(t-Butyl)₂ (S,R-Konfiguration).
L16: Formel XII, R₈ Methyl, X₁ ist -P(C₆H₅)₂ und X₂ ist -P[(3,5-Di(trifluormethyl)-C₆H₃)]₂ (S,R-Konfiguration).
L17: Formel IX, R₇ ist Methoxy, X₁ und X₂ sind je -P(C₆H₅)₂.
L18: Formel X, R₈ ist Methyl, X₁ ist -P(C₆H₅)₂, X₂ ist -P(t-Butyl)₃ (R,S-Konfiguration).
L19: Formel X, R₈ ist Methyl, X₁ ist -P[(3,5-Dimethyl-4-methoxy-C₆H₂)]₂, X₂ ist -P[(3,5-Di(methyl)-C₆H₃)]₂.
L20: Formel X, R₈ ist Methyl, X₁ ist -P(C₆H₁₁)₂, X₂ ist -P(t-Butyl)₂.
L21: Formel IX, R₇ ist Hydroxy, X₁ und X₂ sind je -P(C₆H₅)₂.
L22: Formel XV, R₁₁ ist Phenyl, R₁₂ ist 2-Methoxy-phen-1-yl.
L23: Formel XII, R₈ Methyl, X₁ ist -P(4-Methoxy-C₆H₄)₂, X₂ ist -P(Norbornyl)₂.
L24: Formel XI, R₉ ist H, X₁ und X₂ sind je -P(i-Propyl)₂.
L25: Formel X, R₈ ist Methyl, X₁ und X₂ sind je -P[(3,5-Di(methyl)-C₆H₃)]₂.
L26: Formel VII, R₃ und R₄ sind zusammen -O-CH₂CH₂-N(CH₃)-, X₁ und X₂ sind je - P(C₆H₅)₂.

### Beispiel B1: Hydrierung mit Rutheniumkomplexen

Ein Autoklav wurde bei einem angelegten Druck von 10-12 bar mit Argon gefüllt und wieder entladen. Diese Operation wurde viermal durchgeführt. Nun wurden 0,277 g (1,0 mmol) cis-1 in einem 10 ml-Schlenkgefäss mit Magnetrührer plaziert und dieses sechsmal einer Sequenz aus Anlegen von einem hohem Vakuum und entspannen mit Argon ausgesetzt. Dann wurden 5 ml frisch destilliertes Methanol zugegeben. Zu der gebildeten Lösung wurden anschliessend vorsichtig 60 µl Salzsäure (1 N) zugegeben. In einem weiterem, nach obigem Verfahren unter Argon gesetztem, 10 ml-Schlenkgefäss wurden Metallkomplex (0,005 mmol) und Ligand (0,0106 mmol) gegeben und in 5 ml Methanol gelöst. Beide Lösungen wurden für 10 Minuten bei Raumtemperatur gerührt und anschliessend mittels Kanülen und leichtem Argonstrom in den Autoklaven überführt. Der Autoklav wurde mit Wasserstoffgas gefüllt (10 bar, fünfmal) und schliesslich Wasserstoffgas bis zum gewünschten Druck aufgepresst. Dann wurde die gewünschte Reaktionstemperatur eingestellt und der Rührer gestartet. Nach 16 Stunden Reaktionszeit wurde der Druck auf Normaldruck entspannt und die Reaktionsmischung auf Raumtemperatur abgekühlt. Es wurde eine klare Lösung erhalten, die nach der erwähnten Methode chromatographisch analysiert wird. Die Ergebnisse sind in Tabelle 7 zusammengefasst.

**Tabelle 7:**

| Metallkomplex | Ligand (Konfiguration) | Additiv (ml) | Lösungsmittel | Druck 10⁶ Pa | T (°C) | Umsatz (%) | Selektivität |
|---|---|---|---|---|---|---|---|
| [RuCl₂(S,S-dpen)] | L1 | 0,3^{a)} | iPrOH | 2 | 25 | 100 | >99,5:<0,5 |
| [RuCl₂(R-daipen)] | L1 | 0,3^{a)} | iPrOH | 2 | 25 | 100 | 96,7:3,3 |
| [RuCl₂(P(C₆H₅) ₃)₃] | L2 (S,S) | 0,5^{b)} | Tol | 5 | 25 | 100 | 92,5:7,5 |
| [RuCl₂(P(C₆H₅) ₃)₃] | L2 (S,S) | 1,0^{b)} | Tol | 2 | 25 | 100 | 92,5:7,5 |
| [RuCl₂(P(C₆H₅)₃)₃] | L3 (R,R) | 1,0^{b)} | Tol | 2 | 25 | 100 | 90:10 |
| [RuCl₂(P(C₆H₅) ₃)₃] | L3 (S,S) | 1,0^{b)} | Tol | 2 | 25 | 100 | 90:10 |
| [RuCl₂(P(C₆H₅) ₃)₃] | L4 (S,S) | 1,0^{b)} | Tol | 2 | 25 | > 99 | 87:13 |
| [RuCl₂(P(C₆H₅) ₃)₃] | L5 (S,S) | 1,0^{b)} | Tol | 2 | 25 | > 98,5 | 75:25 |
| [Rul₂(p-Cumol)]₂ | L6 (S) | 0,06^{c)} | MeOH | 8 | 70 | > 93 | 92,5:7,5 |
| [Rul₂(p-Cumol)]₂ | L7 (R) | -- | MeOH | 8 | 70 | > 97 | 92:8 |
| [Rul₂(p-Cumol)]₂ | L8 (R,R) | -- | MeOH | 9 | 70 | 20 | 90:10 |
| [Rul₂(p-Cumol)]₂ | L9 (R,R) | -- | MeOH | 9 | 50 | 40 | 89:11 |
| [Rul₂(p-Cumol)]₂ | L10 (R,S) | -- | MeOH | 8 | 60 | 50 | 89:11 |
| [Rul₂(p-Cumol)]₂ | L6 (R) | 0,06^{c)} | MeOH | 8 | 70 | 92 | 87,4:12,6 |
| [Rul₂(p-Cumol)]₂ | L11 | 0,06^{c)} | MeOH | 8 | 70 | 100 | 87:13 |
| [Rul₂(p-Cumol)]₂ | L12 (R,R) | -- | MeOH | 9 | 50 | 35 | 86:14 |
| [Rul₂(p-Cumol)]₂ | L13(R,S) | -- | MeOH | 9 | 50 | 48 | 86:14 |
| [Rul₂(p-Cumol)]₂ | L14 (R,R) | -- | MeOH | 9 | 50 | 65 | 86:14 |
| [Rul₂(p-Cumol)]₂ | L15 (S,R) | -- | MeOH | 9 | 60 | 60 | 85:15 |
| [Rul₂(p-Cumol)]₂ | L16 (S,R) | -- | MeOH | 9 | 70 | 80 | 84:16 |
| [Rul₂(p-Cumol)]₂ | L17 (S,S) | -- | MeOH | 8 | 80 | 99 | 83:17 |
| [Rul₂(p-Cumol)]₂ | L18 (R,S) | -- | MeOH | 9 | 60 | 50 | 81:19 |
| [Rul₂(p-Cumol)]₂ | L19 (R,S) | -- | MeOH | 8 | 70 | 80 | 80:20 |

| | | | | | | | |
|---|---|---|---|---|---|---|---|
| ^{a)} Kalium-t-butylat (1M); ^{b)} NaOH (1N); ^{c)} Salzsäure (1 N). | | | | | | | |

Das Molverhältnis (mol/mol) von cis-1 zu Katalysator betrug 200 (1 mmol cis-1 zu 0,005 mmol Katalysator (precursor).

### Beispiel B2: Hydrierung mit Rhodiumkomplexen

Entsprechend der Verfahrensvorschrift gemäß Beispiel B1 wurden Hydrierungen mit Rhodiumkomplexen durchgeführt.

Die Abkürzungen bedeuten: nbd = Norbornadien, cod = Cyclooctadien. Das Additiv Methansulfonsäure (MsOH) wird in einer Menge von 0,5 Äquivalenten pro Äquivalent cis-1 zugegeben. Die Ergebnisse sind in Tabelle 8 aufgeführt.

**Tabelle 8:**

| Metallkomplex | Ligand (Konfiguration) | Additiv (ml) | Lösungsmittel | Druck 10⁶ Pa | T (°C) | Umsatz (%) | Selektivität |
|---|---|---|---|---|---|---|---|
| [Rh(nbd)₂]BF₄ | L15 (R,S) | -- | MeOH | 9 | 70 | 100 | 89,6:10,4 |
| [Rh(nbd)₂]BF₄ | L11 (R) | MsOH | MeOH | 9 | 60 | 100 | 82:18 |
| [Rh(nbd)Cl]₂ | L20 (R,S) | -- | Tol | 9 | 70 | 100 | 82:18 |
| [Rh(nbd)₂]BF₄ | L21 (S,S) | -- | MeOH | 5 | 25 | 68 | 80:20 |
| [Rh(cod)L22] BF₄ | L22 (S,S) | -- | MeOH | 8 | 50 | 70 | 79:21 |
| [Rh(nbd)₂]BF₄ | L23 (R) | -- | MeOH | 9 | 50 | 93 | 79:21 |
| [Rh(cod)L24] BF₄ | L24 | MsOH | MeOH | 5 | 50 | 100 | 73,5:26,5 |

### Beispiel B3: Hydrierung mit Iridiumkomplexen

Entsprechend der Verfahrensvorschrift gemäß Beispiel B1 wurden Hydrierungen mit Iridiumkomplexen durchgeführt. Die Reaktionszeit beträgt 18 Stunden. Das Additiv Methansulfonsäure (MsOH) wird in einer Menge von 0,5 Äquivalenten pro Äquivalent cis-1 zugegeben. Die Lösungsmittelmenge ist 10 ml. Die Ergebnise sind in Tabelle 9 aufgeführt.

**Tabelle 9:**

| Metallkomplex | Ligand (Konfiguration) | Additiv (ml) | Lösungsmittel | Druck 10⁶ Pa | T (°C) | Umsatz (%) | Selektivität |
|---|---|---|---|---|---|---|---|
| [Ir(cod)Cl]₂ | L6 (R) | -- | EtOH | 5 | 25 | 80 | 83,5:16,5 |
| [Ir(cod)Cl]₂ | L25 (R,S) | -- | EtOH/Tol ^{a)} | 5 | 25 | 50 | 83,5:16,5 |
| [Ir(cod)Cl]₂ | L26 (R) | MsOH | EtOH | 5 | 25 | 30 | 74,5:25,5 |

| | | | | | | | |
|---|---|---|---|---|---|---|---|
| ^{a)} Volumenverhältnis 1:1 | | | | | | | |

### C) Hydrierung mit Metallhydriden

### Beispiel C1:

0,1 g cis-1 (0,36 mmol) wurden in einen 10 ml Schlenkkolben gegeben und anschliessend der Kolben durch Anlegen von Vakuum und Spülen mit Argon (insgesamt dreimal) inertisiert. Dann wurden 1,7 ml über einem Molekularsieb gefiltertes Tetrahydrofuran (THF) zugegeben. Die gelbe Lösung wurde auf die gewünschte Temperatur gekühlt. Bei dieser Temperatur wurden 0,36 ml 1 M Lösung Metallhydrid in THF (0,36 mmol) über einen Zeitraum von 15 Minuten zugefügt. Nach Beendigung der Reaktion lässt man auf Raumtemperatur erwärmen und untersucht die Reaktionsmischung chromatographisch. Die Ergebnisse sind in Tabelle 10 angegeben. Li- beziehungsweise Na-Selectride ist Lithium- beziehungsweise Natrium-tris-sekundärbutylborhydrid. LiBH ist Lithium-tris-isoamylborhydrid.

**Tabelle 10:**

| Metallhydrid | Lösungsmittel | T(°C) | Zeit (h) | cis-1 | III/IV | V/VI | SAN | Selektivität |
|---|---|---|---|---|---|---|---|---|
| (Äquivalente) | | | | (%) | (%) | (%) | (%) | |
| Li-Selectride (1) | THF | -78 | 1 | 0 | 98 | < 0,5 | 2 | >99,5:<0 ,5 |
| Li-Selectride (1) | THF | -20 | 1 | 0 | 90 | < 0,5 | 10 | >99,5:<0 ,5 |
| Li-Selectride (1) | THF | -12 | 0,17 | 0 | 95 | < 0,5 | 5 | >99,5:<0 ,5 |
| Li-Selectride (0,8) | THF | -12 | 0,17 | 2 | 89 | < 0,5 | 9 | >99,5:<0 ,5 |
| Na-Selectride (1) | THF | -15 | 1 | 0 | 90 | < 0,5 | 10 | >99,5:<0 ,5 |
| LiBH (1) | THF | -15 | 1 | 0 | 84 | 0 | 16 | >99,5:<0 ,5 |

## Patentansprüche

1. Verfahren zur Herstellung von Verbindungen der Formeln 111 oder IV oder Mischungen davon, 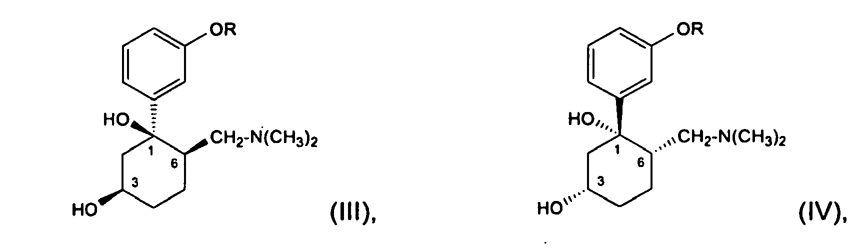 worin R für Wasserstoff oder C₁-C₄-Alkyl steht, durch Hydrierung der Ketogruppe von Verbindungen der Formeln I oder II oder Mischungen davon, 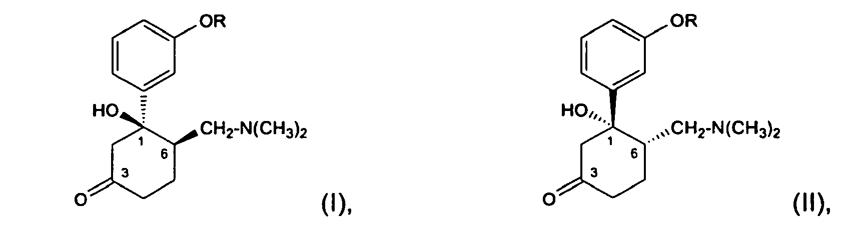 **dadurch gekennzeichnet, dass** die Hydrierung
a) mit Wasserstoff in Gegenwart von Platindioxid, Platin(IV)chlorid, Platin(II)chlorid oder Raney-Nickel in heterogener Phase;
b) mit Wasserstoff in Gegenwart eines Rhodium-, Iridium- oder Rutheniumkomplexes mit wenigstens einem chiralen Diphosphin-Liganden, Diamin-Liganden oder Phosphinit-Liganden in homogener Phase; oder
c) mit einem Alkalimetall-trialkylborhydrid oder einem Alkalimetalltrialkylaluminiumhydrid
durchgeführt wird.

2. Verfahren gemäß Anspruch 1, **dadurch gekennzeichnet, dass** R Wasserstoff oder Methyl darstellt.

3. Verfahren gemäß Anspruch 1 oder 2, **dadurch gekennzeichnet, dass** das Verhältnis von Diastereomeren der Formeln III und IV zu Diastereomeren der Formeln V und VI 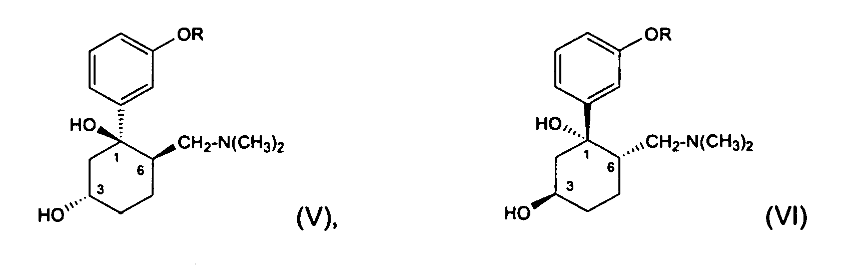 mindestens 75:25 beträgt.

4. Verfahren gemäß einem oder mehreren der Ansprüche 1-3, **dadurch gekennzeichnet, dass** der Wasserstoffdruck bei den Verfahrensvarianten a) und b) von 10⁵ bis 2x10⁷ Pa (Pascal) beträgt.

5. Verfahren gemäß einem oder mehreren der Ansprüche 1-4, **dadurch gekennzeichnet, dass** die Temperatur von -80 bis 150 °C beträgt.

6. Verfahren gemäß einem oder mehreren der Ansprüche 1-5, **dadurch gekennzeichnet, dass** das Platindioxid in Mengen von 0,1 bis 15 Gew.-%, das Raney-Nickel in Mengen von 1 bis 50 Gew.-%, die Rhodium-, Iridium- oder Rutheniumkomplexe mit chiralen Diphosphinen in Mengen von 0,0001 bis 10 Gew.-%, und die Alkalimetall-trialkylborhydride und Alkalimetalltrialkylaluminiumhydrid in Mengen von 0,5 bis 1,5 Äquivalenten eingesetzt werden, jeweils bezogen auf die Verbindungen der Formeln I und/oder II.

7. Verfahren gemäß einem oder mehreren der Ansprüche 1-6, **dadurch gekennzeichnet, dass** die Hydrierung mit Platindioxid in Alkoholen, bevorzugt Methanol, Ethanol oder deren Mischungen, und die Hydrierung mit Raney-Nickel in Alkoholen, Carbonsäureestern, aromatischen Kohlenwasserstoffen oder Mischungen aus wenigstens zwei dieser Lösungsmittel durchgeführt wird.

8. Verfahren gemäß einem oder mehreren der Ansprüche 1-7, **dadurch gekennzeichnet, dass** der Diphoshin-Ligand der Formel Sekundärphosphin-Gerüst-Sekundärphosphin entspricht, der Ligand mit dem Metallatom einen fünf- bis zehngliedrigen Ring bildet, und das Gerüst 2 bis 30 C-Atome und gegebenenfalls Heteroatome, die unabhängig voneinander ausgewählt sind aus der Gruppe bestehend aus O, S, NH und N-C₁-C₄-Alkyl, sowie ggf. Übergangsmetallatome enthält.

9. Verfahren gemäß Anspruch 8, **dadurch gekennzeichnet, dass** es sich bei dem Gerüst um bivalente Reste von Alkanen, Heteroalkanen, Alkenen, Zykloalkanen, Zykloalkenen, Heterozykloalkanen, Heterozykloalkenen, Bizykloalkanen, Bizykloheteroalkanen, Spirobiszykloalkanen, Spirobiszykloheteroalkanen, Arylenen, Heteroarylenen, Bisrarylenen, Bisheteroarylenen, Metallocenen, bevorzugt Ferrocen handelt, und der Rest unsubstituiert oder einfach oder mehrfach substituiert ist, wobei die Substituenten ausgewählt sind aus der Gruppe bestehend aus C₁-C₆-Alkyl, C₁-C₆-Alkoxy, C₁-C₆-Alkylthio, C₄-C₈-Cycloalkyl, Phenyl, Benzyl, Phenoxy, Benzyloxy, Phenylthio, Benzylthio, Halogen, OH, Tri(C₁-C₆-Alkyl)silyl, Sekundäramino, -CO₂H, -SO₃H, -CO₂R', -SO₃R', -O-C(O)-R', -NH-C(O)R', -O-SO₃-R' und -NH-SO₃R', wobei R' für C₁-C₆-Alkyl, C₄-C₈-Cycloalkyl, Phenyl oder Benzyl steht.

10. Verfahren gemäß Anspruch 8 oder 9, **dadurch gekennzeichnet, dass** die sekundären Phosphingruppen gleiche oder verschiedene Kohlenwasserstoffreste als Substituenten enthalten und die beiden sekundären Phosphingruppen im Diphosphinliganden gleich oder verschieden sind.

11. Verfahren gemäß einem oder mehreren der Ansprüche 8-10, **dadurch gekennzeichnet, dass** die zwei Sekundärphosphinoreste unabhängig voneinander nicht-zyklisches Sekundärphosphin ausgewählt aus der Gruppe -P(C₁-C₆-Alkyl)₂, -P(C₅-C₈-Cycloalkyl)₂, -P(C₇-C₈-Bicycloalkyl)₂, -P(o-Furyl)₂, -P(C₆H₅)₂, -P[2-(C₁-C₆-Alkyl)C₆H₄]₂, -P[3-(C₁-C₆-Alkyl)C₆H₄]₂, -P[4-(C₁-C₆-Alkyl)C₆H₄]₂, -P[2-(C₁-C₆-Alkoxy)C₆H₄]₂, -P[3-(C₁-C₆-Alkoxy)C₆H₄]₂, -P[4-(C₁-C₆-Alkoxy)C₆H₄]₂, -P[2-(Trifluormethyl)C₆H₄]₂, -P[3-(Trifluormethyl)C₆H₄]₂, -P[4-(Triftuormethy)C₆H₄]₂,-P[3,5-Bis(trifluormethyl)C₆H₃]₂, -P[3,5-Bis(C₁-C₆-Alkyl)₂C₆H₃]₂, -P[3,5-Bis(C₁-C₆-Alkoxy)₂C₆H₃]₂, und -P[3,5-Bis(C₁-C₆-Alky)₂-4-(C₁-C₆-Alkoxy)C₆H₂]₂. oder zyklisches Phosphin, ausgewählt aus der Gruppe 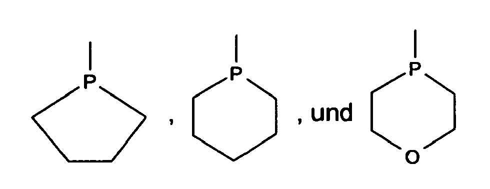 bedeuten, die jeweils unsubstituiert oder ein- oder mehrfach substituiert sind mit C₁-C₄-Alkyl, C₁-C₄-Alkoxy, C₁-C₄-Alkoxy-C₁-C₂-Alkyl, Phenyl, Benzyl, Benzyloxy, oder C₁-C₄-Alkyliden-dioxyl.

12. Verfahren gemäß einem oder mehreren der Ansprüche 8-11, **dadurch gekennzeichnet, dass** es sich bei den Diphosphinliganden um solche der Formeln VII bis XV handelt: 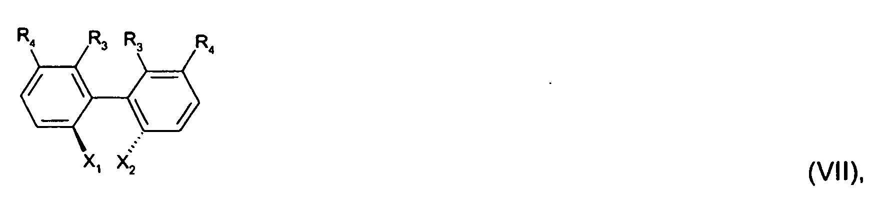 worin X₁ und X₂ für gleiches oder verschiedenes Sekundärphosphino steht, R₃ C₁-C₄-Alkoxy oder C₁-C₄-Alkyl bedeutet, R₄ Wasserstoff ist oder die gleiche Bedeutung wie R₃ hat, oder R₃ und R₄ zusammen mit den C-Atomen, an die sie gebunden sind, einen fünf- oder sechsgliedrigen carbozyklischen Ring oder einen heterozyklischen Ring mit Heteroatomen unabhängig voneinander ausgewählt aus der Gruppe O, S, -N= und -N(C₁-C₄-Alkyl) bilden; 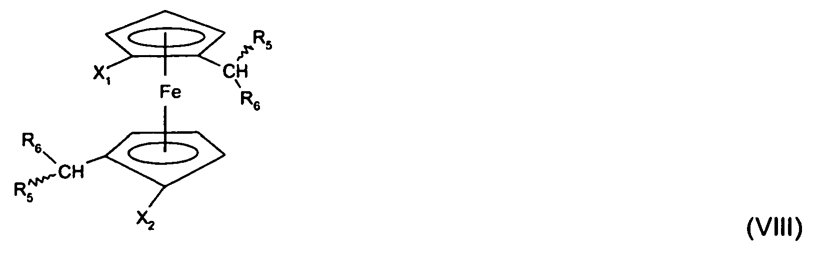 worin X₁ und X₂ für gleiches oder verschiedenes Sekundärphosphino steht, R₆ Sekundäramino bedeutet, und R₅ (C₁-C₆-Alkyl), Cyclohexyl, Phenyl oder Benzyl darstellt; 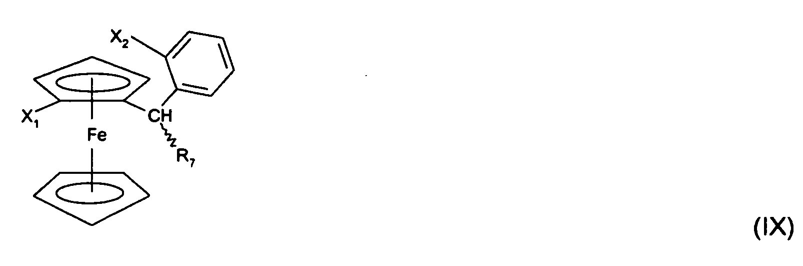 worin X₁ und X₂ für gleiches oder verschiedenes Sekundärphosphino steht und R₇ -OH, C₁-C₁₀-Alkoxy, Phenoxy, Benzyloxy oder C₁-C₈-Acyloxy bedeutet; 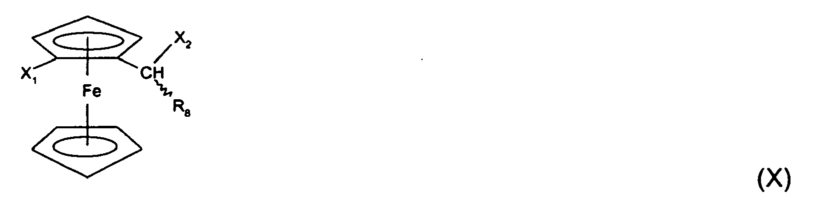 worin X₁ und X₂ für gleiches oder verschiedenes Sekundärphosphino steht und R₈ C₁-C₆-Alkyl, Cyclohexyl, Phenyl oder Benzyl darstellt; 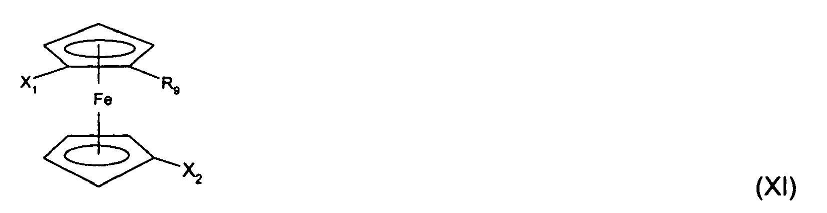 worin X₁ und X₂ für gleiches oder verschiedenes Sekundärphosphino steht und R₉ Wasserstoff bedeutet, oder eine Gruppe -CH(R₇)R₈ darstellt, worin R₇ -OH, C₁-C₁₀-Alkoxy, Phenoxy, Benzyloxy oder C₁-C₈-Acyloxy ist; und R₈ C₁-C₆-Alkyl, Cyclohexyl, Phenyl oder Benzyl bedeutet; 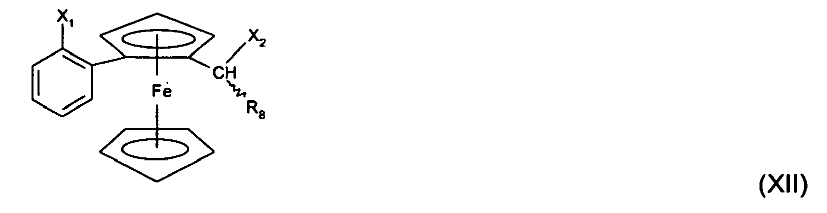 worin X₁ und X₂ für gleiches oder verschiedenes Sekundärphosphino steht und R₈ C₁-C₆-Alkyl, Cyclohexyl, Phenyl oder Benzyl bedeutet; 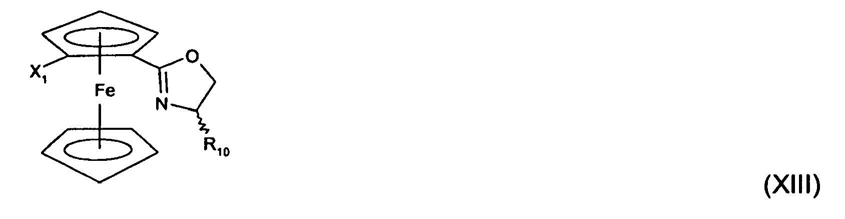 worin X₁ für Sekundärphosphino steht und R₁₀ C₁-C₆-Alkyl, Cyclohexyl, Phenyl oder Benzyl bedeutet; 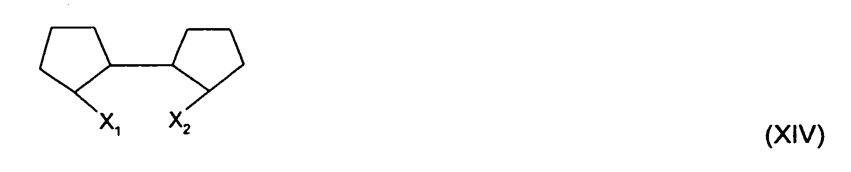 worin X₁ und X₂ für gleiches oder verschiedenes Sekundärphosphino steht; und 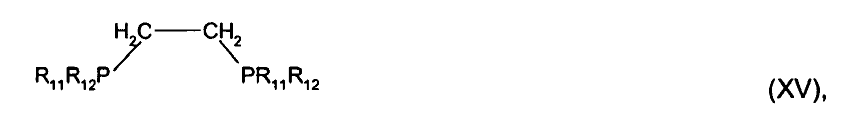 worin R₁₁ und R₁₂ verschiedene Kohlenwasserstoffreste mit 1 bis 20 C-Atomen darstellen, die unsubstituiert oder mit C₁-C₆-Alkyl und/oder C₁-C₆-Alkoxy substituiert sind.

13. Verfahren gemäß Anspruch 12, **dadurch gekennzeichnet, dass** die Liganden L1-L26 eingesetzt werden, die definiert sind wie folgt:
L1: Formel VII, R₃ und R₄ sind zusammen -CH=CH-CH=CH-, X₁ und X₂ sind je - P(C₆H₅)₂;
L2: Formel XIII, R₁₀ ist tert-Butyl, X₁ ist -P(C₆H₅)₂;
L3: Formel XIII, R₁₀ ist iso-Propyl, X₁ ist -P(C₆H₅)₂;
L4: Formel XIII, R₁₀ ist iso-Propyl, X₁ ist -P[(3,5-Dimethy)-4-methoxyC₆H₂)]₂;
L5: Formel XIII, R₁₀ ist Phenyl, X₁ ist -P(C₆H₅)₂;
L6: Formel VII, R₃ und R₄ sind zusammen -O-CH₂CH₂-N(CH₃)-, X₁ und X₂ sind je -P[(3.5-Dimethy)-C₆H₃)]₂;
L7: Formel VII, R₃ und R₄ sind zusammen -O-CF₂-O-, X₁ und X₂ sind je - P(C₆H₅)₂;
L8: Formel XII, R₈ Methyl, X₁ ist -P(C₆H₅)₂, X₂ ist -P[(3,5-Di(trifluormethyl)-C₆H₃)]₂ (R,R-Konfiguration);
L9: Formel XIV, X₁ und X₂ sind je -P(C₆H₅)₂;
L10: Formel X, R₈ ist Methyl, X₁ ist -P[(3,5-Dimethyt-C₆H₃)]₂, X₂ ist -P[(3,5-Di(trifluormethyl)-C₆H₃)]₂;
L11: Formel VII, R₃ und R₄ sind zusammen -O-CH₂CH₂-N(CH₃)-, X₁ und X₂ sind je -P[(3,5-Dimethyl-4-methoxy-C₆H₂)]₂;
L12: Formel XII, R₈ ist Methyl, X₁ ist -P[(3,5-Dimethyl-4-methyl-C₆H₂)]_{2,} X₂ ist-P[(3,5-Di(trifluormethyl)-C₆H₃)]₂;
L13: Formel VIII, R₅ ist Phenyl und R₆ ist Dimethylamino, X₁ und X₂ sind je-P[(3,5-Dimethyl-4-methoxy-C₆H₂)]₂;
L14: Formel XII, R₈ Methyl, X₁ und X₂ sind je -P(C₆H₅)₂;
L15: Formel X, R₈ ist Methyl, X₁ ist -P(C₆H₅)₂, X₂ ist -P(t-Butyl)₂ (S,R-Konfiguration);
L16: Formel XII, R₈ Methyl, X₁ ist -P(C₆H₅)₂ und X₂ ist -P[(3,5-Di(trifluormethyl)-C₆H₃)]₂ (S,R-Konfiguration);
L17: Formel IX, R₇ ist Methoxy, X₁ und X₂ sind je -P(C₆H₅)₂;
L18: Formel X, R₈ ist Methyl, X₁ ist -P(C₆H₅)₂, X₂ ist -P(t-Butyl)₃ (R,S-Konfiguration);
L19: Formel X, R₈ ist Methyl, X₁ ist -P[(3,5-Dimethy)-4-methoxy-C₆H₂)]₂, X₂ ist - P[(3,5-Di(methyl)-C₆H₃)]₂;
L20: Formel X, R₈ ist Methyl, X₁ ist -P(C₆H₁₁)₂, X₂ ist -P(t-Butyl)₂;
L21: Formel IX, R₇ ist Hydroxy, X₁ und X₂ sind je -P(C₆H₅)₂;
L22: Formel XV, R₁₁ ist Phenyl, R₁₂ ist 2-Methoxy-phen-1-yl;
L23: Formel XII, R₈ Methyl, X₁ ist -P(4-Methoxy-C₆H₄)₂, X₂ ist -P(Norbornyl)₂;
L24: Formel XI, R₉ ist H, X₁ und X₂ sind je -P(i-Propyl)₂;
L25: Formel X, R₈ ist Methyl, X₁ und X₂ sind je -P[(3,5-Di(methyl)-C₆H₃)]₂;
L26: Formel VII, R₃ und R₄ sind zusammen -O-CH₂CH₂-N(CH₃)-, X₁ und X₂ sind je -P(C₆H₅)₂.

14. Verfahren gemäß einem oder mehreren der Ansprüche 1-13, **dadurch gekennzeichnet, dass** Rutheniumkomplexe des Typs [RuHalogenid₂(Diphosphin Formel Vll)(chirales Diamin)] oder des Typs [RuHalogenid₂(Ligand Formel XIII)(tertiäres Phosphin)] als homogene und chirale Hydrierkatalysatoren verwendet werden, wobei Halogenid bevorzugt Cl, Br oder I ist.

15. Verfahren gemäß einem oder mehreren der Ansprüche 1-6, **dadurch gekennzeichnet, dass** die Alkalimetall-trialkylborhydride und Alkalimetall-trialkylaluminiumhydride den Formeln XXI und XXIa entsprechen,
Alkalimetall[B(R₁₃)₃H] (XXI),
Alkalimetall[Al(R₁₃)₃H] (XXIa),
worin Alkalimetall für Li, Na oder K steht und R₁₃ ein linearer oder verzweigter Alkylrest mit 1 bis 18, bevorzugt 3 bis 18 und besonders bevorzugt 4 bis 12 C-Atomen ist.

16. Verfahren gemäß Anspruch 15, **dadurch gekennzeichnet, dass** das Alkyl in α-Stellung verzweigt ist.

17. Verfahren gemäß Anspruch 15 oder 16, **dadurch gekennzeichnet, dass** es sich bei R₁₃ um einen Rest ausgewählt aus der Gruppe bestehend aus But-2-yl, Pent-2-yl, Hex-2-yl, Hept-2-yl, Oct-2-yl, 2- und 1,2-Dimethyl-but-1-yl, 2-Ethylpent-1-yl, Hex-2-yl, 2-Methyl- oder 2-Ethyl-hex-1-yl, 1,2,2-Trimethyl-eth-1-yl und 1,2-Dimethyl-but-1-yl handelt.

## Claims

1. Method for producing compounds of formulae III or IV or mixtures thereof 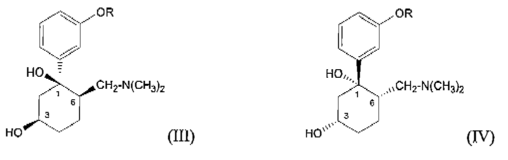 wherein R represents hydrogen or C₁-C₄ alkyl, by hydrogenation of the keto group of compounds of formulae I or II or mixtures thereof 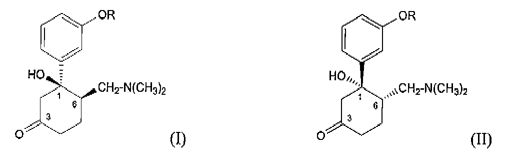 **characterised in that** the hydrogenation is conducted
a) with hydrogen in the presence of platinum dioxide, platinum(IV) chloride, platinum(II) chloride or Raney nickel in heterogeneous phase;
b) with hydrogen in the presence of a rhodium, iridium or ruthenium complex with at least one chiral diphosphine ligand, diamine ligand or phosphinite ligand in homogeneous phase; or
c) with an alkali metal trialkyl boron hydride or an alkali metal trialkyl aluminium hydride.

2. Method according to claim 1, **characterised in that** R represents hydrogen or methyl.

3. Method according to claim or 2, **characterised in that** the ratio of diastereomers of formulae III and IV to diastereomers of formulae V and VI 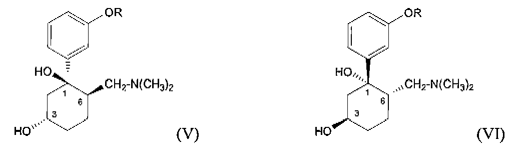 amounts to at least 75:25.

4. Method according to one or more of claims 1-3, **characterised in that** the hydrogen pressure in the case of method variants a) and b) ranges from 10⁵ to 2x10⁷Pa (Pascal).

5. Method according to one or more of claims 1-4, **characterised in that** the temperature ranges from -80° to 150°C.

6. Method according to one or more of claims 1-5, **characterised in that** platinum dioxide is used in quantities of 0.1 to 15% by wt., Raney nickel in quantities of 1 to 50% by wL, the rhodium, iridium or ruthenium complexes with chiral diphosphines in quantities of 0.0001 to 10% by wt. and alkali metal trialkyl boron hydrides and alkali metal trialkyl aluminium hydride in quantities of 0.5 to 1.5 equivalents, respectively calculated on the basis of the compounds of formulae I and/or 11.

7. Method according to one or more of claims 1-6, **characterised in that** the hydrogenation with platinum dioxide is conducted in alcohols, preferably methanol, ethanol or mixtures thereof, and the hydrogenation with Raney nickel is conducted in alcohols, carboxylic acid esters, aromatic hydrocarbons or mixtures of at least two of these solvents.

8. Method according to one or more of claims 1-7, **characterised in that** the diphosphine ligand of the formula corresponds to secondary phosphine-skeleton-secondary phosphine, the ligand with the metal atom forms a five- to ten-membered ring, and the skeleton contains 2 to 30 C atoms and possibly heteroatoms, which are selected independently of one another from the group comprising O, S, NH and N-C₁-C₄ alkyl, and also possibly transition metal atoms.

9. Method according to claim 8, **characterised in that** the skeleton comprises bivalent residues or alkanes, heteroalkanes, alkenes, cycloalkanes, cycloalkenes, heterocycloalkanes, heterocycloalkenes, bicycloalkanes, bicycloheteroalkanes, spirobiscykloalkanes, spirobiscycloheteroalkanes, arylenes, heteroarylenes, bisarylenes, bisheteroarylenes, metallocenes, preferably ferrocenes, and the rest is unsubstituted or mono- or polysubstituted, wherein the substituents are selected from the group comprising C₁-C₆ alkyl, C₁-C₆, alkoxy, C₁-C₆ alkylthio, C₄-C₈-cycloalkyl, phenyl, benzyl, phenoxy, benzyloxy, phenylthio, benzylthio, halogen, OH, tri(C₁-C₆, alkyl)silyl, secondary amino, -CO₂H, -SO₃H, -CO₂R', -SO₃R', -O-C(O)-R', -NH-C(O)R', -O-SO₃-R' and -NH-SO₃R', wherein R' represents C₁-C₆, alkyl, C₄-C₈ cycloalkyl, phenyl or benzyl.

10. Method according to claim 8 or 9, **characterised in that** the secondary phosphine groups contain the same or different hydrocarbon residues and the two secondary phosphine groups in the diphosphine ligand are the same or different.

11. Method according to one or more of claims 8-10, **characterised in that** the two secondary phosphine residues represent non-cyclic secondary phosphine selected independently of one another from the group -P(C₁-C₆ alkyl)₂, -P(C₅-C₈ cycloalkyl)₂, -P(C₇-C₈ bicycloalkyl)₂, -P(o-furyl)₂, -P(C₆H₅)₂, -P[2-(C₁-C₆ alkyl)C₆H₄]₂, -P[3-(C₁-C₆ alkyl)C₆H₄]₂, -P[4-(C₁-C₆ alkyl)C₆H₄]₂, -P[2-(C₁-C₆ alkoxy)C₆H₄]₂, -P[3-(C₁-C₆ alkoxy)C₆H₄]₂, -P[4-(C₁-C₆ alkoxy)C₆H₄]₂, -P[2-(trifluoromethyl)C₆H₄]₂, -P[3-(trifluorometliyl)C₆H₄]₂, -P[4-(trifluoromethyl)C₆H₄]₂, -P[3,5-bis(tritluoroxnethyl)C₆H₃]₂, -P[3,5-bis(C₁-C₆-alkyl)₂C₆H₃]₂, -P[3,5-bis(C₁-C₆ alkoxy)₂C₆H₃]₂, and -P[3,5-bis(C₁-C₆, alkyl)₂-4-(C₁-C₆-alkoxy)C₆H₂]₂, or cyclic phosphine, selected from the group 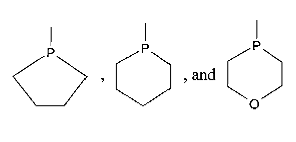 which are respectively unsubstituted or mono- or polysubstituted with C₁-C₄ alkyl, C₁-C₄ alkoxy, C₁-C₄ alkoxy-C₁-C₂ alkyl, phenyl, benzyl, benzyloxy, or C₁-C₄ alkylidene-dioxyl.

12. Method according to one or more of claims 8-11, **characterised in that** the diphosphine ligands are those of formulae VII to XV: 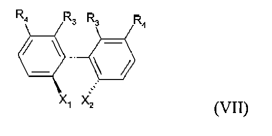 wherein X₁ and X₂ represent the same or different secondary phosphino, R₃ represents C₁-C₄ alkoxy or C₁-C₄ alkyl, R₄ is hydrogen or represents the same as R₃, or R₃ and R₄ together with the C atoms, to which they are bonded, form a five- or six-membered carbocyclic ring or a heterocyclic ring with heteroatoms selected independently of one another from the group O, S, -N= and -N(C₁-C₄ alkyl); 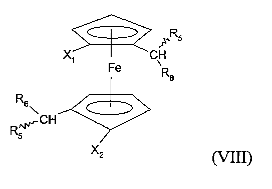 wherein X₁ and X₂ represent the same or different secondary phosphino, R₆ represents secondary amino, and R₅ represents (C₁-C₆ alkyl), cyclohexyl, phenyl or benzyl; 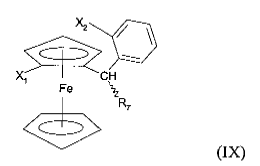 wherein X₁ and X₂ represent the same or different secondary phosphino, and R₇ represents -OH, C₁-C₁₀ alkoxy, phenoxy, benzyloxy or C₁-C₈ acyloxy; 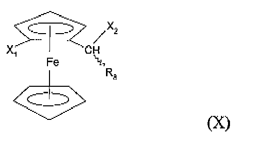 wherein X₁ and X₂ represent the same or different secondary phosphino, and R₈ represents C₁-C₆ alkyl, cyclohexyl, phenyl or benzyl; 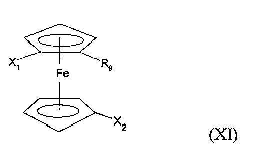 wherein X₁ and X₂ represent the same or different secondary phosphino, and R₉ represents hydrogen or a group -CH(R₇)R₈, wherein R₇ is -OH, C₁-C₁₀ alkoxy, phenoxy, benzyloxy or C₁-C₈ acyloxy; and R₈ represents C₁-C₆ alkyl, cyclohexyl, phenyl or benzyl; 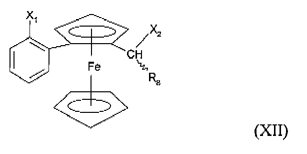 wherein X₁ and X₂ represent the same or different secondary phosphino, and R₈ represents C₁-C₆ alkyl, cyclohexyl, phenyl or benzyl; 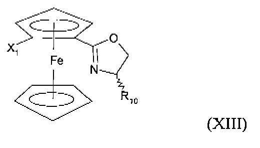 wherein X₁ represents secondary phosphino and R₁₀ represents C₁-C₆ alkyl, cyclohexyl, phenyl or benzyl; 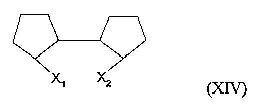 wherein X₁ and X₂ represent the same or different secondary phosphino; and 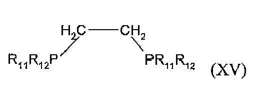 wherein R₁₁ and R₁₂ represent different hydrocarbon residues with 1 to 20 C atoms, which are unsubstituted or are substituted with C₁-C₆ alkyl and/or C₁-C₆ alkoxy.

13. Method according to claim 12, **characterised in that** ligands L1-L26 are used, which arc defined as follow:
L1: formula VII, R₃ and R₄ together are -CH=CH-CH=CH-, X₁ and X₂ are each -P(C₆H₅)₂;
L2: formula XIII, R₁₀ is tert-butyl, X₁ is -P(C₆H₅)₂;
L3: formula XIII, R₁₀ is iso-propyl, X₁ is -P(C₆H₅)₂;
L4: formula XIII, R₁₀ is iso-propyl, X₁ is -P[(3,5-dimethyl-4-methoxyC₆,H₂)]₂;
L5: formula XIII, R₁₀ is phenyl, X₁ is -P(C₆H₅)₂;
L6: formula VII, R₃ and R₄ together are -O-CH₂CH₂-N(CH₃)-, X₁ and X₂ are each -P[(3,5-dimethyl-C₆H₃)]₂;
L7: formula VII, R₃ and R₄ together arc -O-CF₂-O-, X₁ and X₂ are each -P(C₆H₅)₂;
L8: formula XII, R₈ methyl, X₁ is -P(C₆H₅)₂, X₂ is -P[(3,5-di(trifluoromethyl)-C₆H₃)]₂ (R,R-configuration);
L9: formula XIV, X₁ and X₂ are each -P(C₆H₅)₂;
L10: formula X, R₈ is methyl, X₁ is -P[(3,5-dimethyl-C₆H₃)]₂, X₂ is -P[(3,5-di(trifluoroinethyl)-C₆H₃)]₂;
L11: formula VII, R₃ and R₄ together arc -O-CH₂CH₂-N(CH₃)-, X₁ and X₂ arc each -P[(3,5-dimethyl-4-methoxy-C₆H₂)]₂;
L12: formula XII, R₈ is methyl, X₁ is -P[(3,5-dimethyl-4-methyl-C₆H₂)]₂, X₂ is -P[(3 ,5-di(triluoromethyl)-C₆H₃)]₂₁
L13: formula VIII, R₅ is phenyl and R₆ is dimethylamino, X₁ and X₂ are each -P[(3,5-dimethyl-4-methoxy-C₆H₂)]₂;
L14: formula XII, R₈ methyl, X₁ and X₂ are each -P(C₆H₅)₂,
L15: formula X, R₈ is methyl, X₁ is -P(C₆H₅)₂, X₂ is -P(t-butyl)₂ (S,R-configuration);
L16: formula XII, R₈ methyl, X₁ is -P(C₆H₅)₂ and X₂ is -P[(3,5-di(trifluorometliyl)-C₆H₃)]₂ (S,R-configuration);
L17: formula IX, R₇ is methoxy, X₁ and X₂ arc each -P(C₆H₅)₂;
L18: formula X, R₈ is methyl, X₁ is -P(C₆H₅)₂, X₂ is -P(t-butyl)₃ (R,S-configuration);
L19: formula X, R₈ is methyl, X₁ is -P[(3,5-dimethyl-4-methoxy-C₆H₂)]₂, X₂ is -P[(3,5-di(methyl)-C₆H₃)]₂;
L20: formula X, R₈ is methyl, X₁ is -P(C₆H₁₁)₂, X₂ is -P(t-butyl)₂;
L21: formula IX, R₇ is hydroxy, X₁ and X₂ arc each -P(C₆H₅)₂;
L22: formula XV, R₁₁ is phenol, R₁₂ is 2-methoxy-phen-1-yl;
L23: formula XII, R₈ methyl, X₁ is -P(4-methoxy-C₆H₄)₂, X₂ is -P(norbornyl)₂;
L24: formula XI, R₉ is H, X₁ and X₂ arc each -P(i-propyl)₂;
L25: formula X, R₈ is methyl, X₁ and X₂ are each -P[(3,5-di(methyl)-C₆H₃)]₂;
L26: formula VII, R₃ and R₄ together are -O-CH₂CH₂-N(CH₃)-, X₁ and X₂ are each -P(C₆H₅)₂.

14. Method according to one or more of claims 1-13, **characterised in that** ruthenium complexes of the type [Ru halide₂(diphosphine formula VII)(chiral diamine)] or of the type [Ru halide₂(ligand formula XIII)(tertiary phosphine)] are used as homogeneous and chiral hydrogenating catalysts, wherein halide is preferably Cl, Br or I.

15. Method according to one or more of claims 1-6, **characterised in that** the alkali metal trialkyl boron hydrides and alkali metal aluminium hydrides correspond to formulae XXI and XXIa
alkali metal[B(R₁₃)₃H] (XXI)
alkali metal[Al(R₁₃)₃H] (XXIa)
wherein alkali metal represents Li, Na or K and R₁₃ is a linear or branched alkyl residue with 1 to 18, preferably 3 to 18 and particularly preferred 4 to 12 C atoms.

16. Method according to claim 15, **characterised in that** the alkyl is branched in the α position.

17. Method according to claim 15 or 16, **characterised in that** R₁₃ is a residue selected form the group comprising but-2-yl, pent-2-yl, hex-2-yl, hept-2-yl, oct-2-yl, 2- and 1,2-dimethyl-but-1-yl, 2-etliyl-pent-1-yl, hex-2-yl, 2-methyl- or 2-ethyl-hex-1-yl, 1,2,2-trimethyl-eth-1-yl and 1,2-dimethyl-but-1-yl.

## Revendications

1. Procédé pour la préparation de composés des formules III ou IV ou de mélanges de ceux-ci 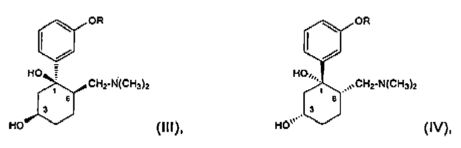 où R représente hydrogène ou C₁-C₄-alkyle, par hydrogénation du groupe céto de composés des formules I ou II ou de leurs mélanges, 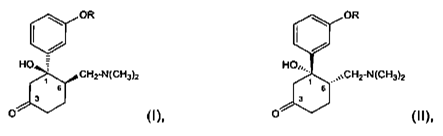 **caractérisé en ce que** l'hydrogénation est réalisée
a) avec de l'hydrogène en présence de dioxyde de platine, de chlorure de platine (IV), de chlorure de platine (II) ou de nickel de Raney en phase hétérogène ;
b) avec de l'hydrogène en présence d'un complexe du rhodium, de l'iridium ou du ruthénium avec au moins un ligand chiral de type diphosphine, diamine ou phosphinite en phase homogène ; ou
c) avec un trialkylborohydrure de métal alcalin ou trialkylaluminohydrure de métal alcalin.

2. Procédé selon la revendication 1, **caractérisé en ce que** R représente hydrogène ou méthyle.

3. Procédé selon la revendication 1 ou 2, **caractérisé en ce que** le rapport de diastéréo-isomères des formules III et IV aux diasléréo-isomères des formules V et VI 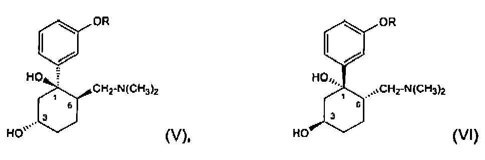 est d'au moins 75:25.

4. Procédé selon l'une ou plusieurs des revendications 1 à 3, **caractérisé en ce que** la pression d'hydrogène dans les variantes de procédé a) et b) est de 10⁵ à 2 x 10' Pa (Pascal).

5. Procédé selon l'une ou plusieurs des revendications 1 à 4, **caractérisé en ce que** la température est de -80 à 150°C.

6. Procédé selon l'une ou plusieurs des revendications 1 à 5, **caractérisé en ce que** le dioxyde de platine est utilisé en des quantités de 0,1 à 15% en poids, le nickel de Raney est utilisé en des quantités de 1 à 50% en poids, les complexes du rhodium, de l'iridium ou du ruthénium avec des diphosphines chorales en des quantités de 0,0001 à 10% en poids, et le trialkylborohydrure de métal alcalin et le trialkylaluminohydrure de métal alcalin en des quantités de 0,5 à 1,5 équivalent, à chaque fois par rapport aux composés des formules I et/ou II.

7. Procédé selon l'une ou plusieurs des revendications 1 à 6, **caractérisé en ce que** l'hydrogénation avec le dioxyde de platine est réalisée dans des alcools, de préférence le méthanol, l'éthanol ou leurs mélanges et l'hydrogénation avec le nickel de Raney est réalisée dans des alcools, des esters d'acide carboxylique, des hydrocarbures aromatiques ou des mélanges d'au moins deux de ces solvants.

8. Procédé selon l'une ou plusieurs des revendications 1 à 7, **caractérisé en ce que** le ligand de type diphosphine correspond à la formule phosphine secondaire-structure-phosphine secondaire, le ligand forme un cycle de cinq à dix chaînons avec l'atome de métal, et la structure contient 2 à 30 atomes de carbone et le cas échéant des hétéroatomes, qui sont choisis indépendamment l'un de l'autre dans le groupe constitué par O, S, NH et N-C₁-C₄-alkyle, ainsi que le cas échéant des atomes de métal de transition.

9. Procédé selon la revendication 8, **caractérisé en ce qu'**il s'agit, pour la structure, de radicaux divalents d'alcanes, d'hétéroalcanes, d'alcènes, de cycloalcanes, de cycloalcènes, d'hétérocycloalcanes, d'hétérocycloalcènes, de bicycloalcanes, de bicyclohétéroalcanes, de spirobiscycloalcanes, de spirobiscyclohétéroalcanes, d'arylènes, d'hétéroarylènes, de bisarylènes, de bishétéroarylènes, de métallocènes, de préférence de ferrocène, et le radical est non substitué ou monosubstitué ou polysubstitué, où les substituants sont choisis dans le groupe constitué par C₁-C₆-alkyle, C₁-C₆-alcoxy, C₁-C₆-alkylthio, C₄-C₈-cycloalkyle, phényle, benzyle, phénoxy, benzyloxy, phénylthio, benzylthio, halogène, OH, tri(C₁-C₆-alkyl)silyle, amino secondaire, -CO₂H, -SO₃H, -CO₂R', -SO₃R', -O-C(O)-R', -NH-C(O)R', -O-SO₃-R' et -NH-SO₃R', où R' représente C₁-C₆-alkyle, C₄-C₈-cycloalkyle, phényle ou benzyle.

10. Procédé selon la revendication 8 ou 9, **caractérisé en ce que** les groupes phosphine secondaire contiennent des radicaux hydrocarbonés identiques ou différents comme substituants et les deux groupes phosphine secondaire dans les ligands de type diphosphine sont identiques ou différents.

11. Procédé selon l'une ou plusieurs des revendications 8 à 10, **caractérisé en ce que** les deux radicaux phosphine secondaire signifient, indépendamment l'un de l'autre, phosphine secondaire non cyclique choisie dans le groupe -P(C₁-C₆-alkyle)₂, -P(C₅-C₈-cycloalkyle)₂, -P(C₇-C₈-bicycloalkyle)₂, -P(O-furyle)₂, -P(C₆H₅)₂, -P[2-(C₁-C₆-alkyl)C₆H₄]₂, -P[3-(C₁-C₆-alkyl)C₆H₄]₂, -P[4-(C₁-C₆-alkyl)C₆H₄]₂, -P[2-(C₁-C₆-alcoxy)C₆H₄]₂, -P[3-(C₁-C₆-alcoxy)C₆H₄]₂, -P[4-(C₁-C₆-alcoxy)C₆H₄]₂, -P[2-(trifluorométhyl)C₆H₄]₂, -P[3-(trifluorométhyl)C₆H₄]₂, -P[4-(trifluorométhyl)C₆H₄]₂, -P[3,5-bis(trifluorométhyl)C₆H₃]₂, -P[3,5-bis(C₁-C₆-alkyl)₂C₆H₃]₂, -P[3,5-bis(C₁-C₆-alcoxy)₂C₆H₃]₂, et -P[3,5-bis(C₁-C₆-alkyl)₂-4-(C₁-C₆-alcoxy)C₆H₂]₂, ou phosphine cyclique, choisie dans le groupe 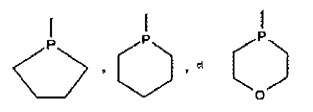 qui sont à chaque fois non substitués ou monosubstitués ou polysubstitués par C₁-C₄-alkyle, C₁-C₄-alcoxy, C₁-C₄-alcoxy-C₁-C₂-alkyle, phényle, benzyle, benzyloxy, ou C₁-C₄-alkylidènedioxyle.

12. Procédé selon l'une ou plusieurs des revendications 8 à 11, **caractérisé en ce qu'**il s'agit, pour les ligands de type diphosphine, de ceux des formules VII à XV : 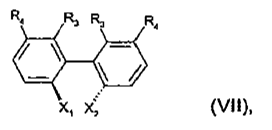 où X₁ et X₂ représentent phosphino secondaire identique ou différent, R₂ signifie C₁-C₄-alcoxy ou C₁-C₄-alkyle, R₄ représente hydrogène ou présente la même signification que R₃, ou R₃ et R₄ forment, ensemble avec les atomes de carbone, auxquels ils sont liés, un cycle carbocyclique de cinq ou six chaînons ou un cycle hétérocyclique avec des hétéroatomes, choisis indépendamment l'un de l'autre dans le groupe 0, S, -N= et -N(C₁-C₄-alkyle) ; 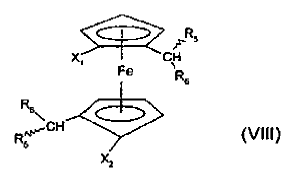 où X₁ et X₂ représentent phosphino secondaire identique ou différent, R₆ signifie amino secondaire, et R₅ représente (C₁-C₆-alkyle), cyclohexyle, phényle ou benzyle ; 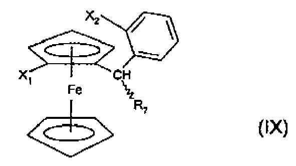 où X₁ et X₂ représentent phosphino secondaire identique ou différent et R₇ signifie -OH, C₁-C₁₀-alcoxy, phénoxy, benzyloxy ou C₁-C₈-acyloxy ; 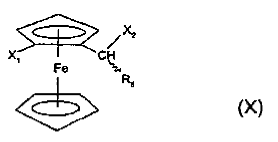 où X₁ et X₂ représentent phosphino secondaire identique ou différent et R₈ signifie C₁-C₆-alkyle, cyclohexyle, phényle ou benzyle ; 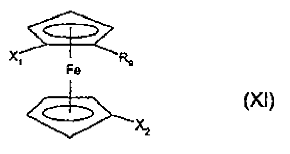 où X₁ et X₂ représentent phosphino secondaire identique ou différent eL R₉ signifie hydrogène, ou représentent un groupe -CH(R₇)R₈, où R₇ représente -OH, C₁-C₁₀-alcoxy, phénoxy, benzyloxy ou C₁-C₈-acyloxy ; et R₈ signifie C₁-C₆-alkyle, cyclohexyle, phényle, ou benzyle ; 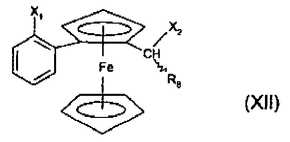 où X₁ et X₂ représentent phosphino secondaire identique ou différent et R₈ signifie C₁-C₆-alkyle, cyclohexyle, phényle ou benzyle ; 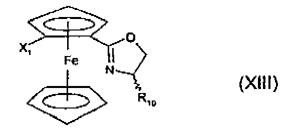 où X₁ représente phosphino secondaire et R₁₀ signifie C₁-C₆-alkyle, cyclohexyle, phényle ou benzyle ; 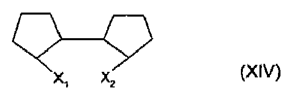 où X₁ et X₂ représentent phosphino secondaire identique ou différent ; et 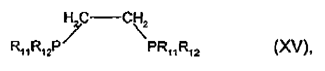 où R₁₁ et R₁₂ représentent des radicaux hydrocarbonés différents comprenant 1 à 20 atomes de carbone, qui sont non substitués ou substitués par C₁-C₆-alkyle et/ou C₁-C₆-alcoxy.

13. Procédé selon la revendication 12, **caractérisé en ce qu'**on utilise les ligands L1-L26 qui sont définis comme suit :
L1 : formule VII, R₃ et R₄ représentent ensemble -CH=CH-CH=CH-, X₁ et X₂ représentent chacun -P(C₆H₅)₂ ;
L2 : formule XIII, R₁₀ représente tert-butyle, X₁ représente -P(C₆H₅)₂ ;
L3 : formule XIII, R₁₀ représente isopropyle, X₁ représente -P(C₆H₅)₂ ;
L4 : formule XIII, R₁₀ représente isopropyle, X₁ représente -P[(3,5-diméthyl-4-méthoxyC₆H₂)]₂ ;
L5 : formule XIII, R₁₀ représente phényle, X₁ représente -P(C₆H₅)₂ ;
L6 : formule VII, R₃ et R₄ représentent ensemble -O-CH₂CH₂-N(CH₃)-, X₁ et X₂ représentent chacun -P[(3,5-diméthyl-C₆H₃)]₂ ;
L7 : formule VII, R₃ et R₄ représentent ensemble -O-CF₂-O-, X₁ et X₂ représentent chacun -P(C₆H₅)₂ ;
L8 : formule XII, R₈ représente méthyle, X₁ représente -P(C₆H₅)₂, X₂ représente -P[(3,5-di(trifluorométhyl)-C₆H₃)]₂ (configuration R,R) ;
L9 : formule XIV, X₁ et X₂ représentent chacun -P(C₆H₅)₂ ;
L10 : formule X, R₈ représente méthyle, X₁ représente -P[(3,5-diméthyl-C₆H₃)]₂, X₂ représente -P[(3,5-di(trifluorométhyl)-C₆H₃)]₂ ;
L11 : formule VII, R₃ et R₄ représentent ensemble -O-CH₂CH₂-N(CH₃)-, X₁ et X₂ représentent chacun -P[(3,5-diméthyl-4-méthoxy-C₆H₂)]₂ ;
L12 : formule XII, R₈ représente méthyle, X₁ représente -P[(3,5-diméthyl-4-méthyl-C₆H₂)]₂, X₂ représente -P[(3,5-di(trifluorométhyl)-C₆-H₃)]₂,
L13 : formule VIII, R₆ représente phényle et R₆ représente diméthylamino, X₁ et X₂ représentent chacun -P[(3,5-diméthyl-4-méthoxy-C₆H₂)]₂ ;
L14 : formule XII, R₈ représente méthyle, X₁ et X₂ représentent chacun -P(C₆H₅)₂ ;
L15 : formule X, R_{θ} représente méthyle, X₁ représente -P(C₆H₅)₂, X₂ représente -P(t-butyle)₂ (configuration S,R) ;
L16 : formule XII, R₈ représente méthyle, X₁ représente -P(C₆H₅) ₂ et X₂ représente -P[(3,5-di(trifluorométhyl)-C₆H₃)]₂ (configuration S,R) ;
L17 : formule IX, R₇ représente méthoxy, X₁ et X₂ représentent chacun -P(C₆H₅)₂ ;
L18 : formule X, R₈ représente méthyle, X₁ représente -P(C₆H₅)₂, X₂ représente -P(t-butyle)₃ (configuration R,S) ;
L19 : formule X, R₈ représente méthyle, X₁ représente -P[(3,5-diméthyl-4-méthoxy-C₆H₂)]₂, X₂ représente-P[(3,5-d1(méthyl)-C₆H₃)]₂ ;
L20 : formule X, R₈ représente méthyle, X₁ représente -P(C₆H₁₁)₂ X₂ représente -P(t-butyle)₂,
L21 : formule IX, R₇ représente hydroxy, X₁ et X₂ représentent chacun -P(C₆H₅)₂ ;
L22 : formule XV, R₁₁ représente phényle, R₁₂ représente 2-méthoxyphén-1-yle,
L23 : formule XII, R₆ représente méthyle, X₁ représente -P(4-méthoxy-C₆H₄)₂, X₂ représente -P(norbornyle)₂ ;
L24 : formule XI, R₉ représente H, X₁ et X₂ représentent chacun -P(i-propyle)₂ ;
L25 : formule X, R₈ représente méthyle, X₁ et X₂ représentent chacun -P[(3,5-di(méthyl)-C₆H₃)]₂ ;
L26 : formule VII, R₃ et R₄ représentent ensemble -0-CH₂CH₂-N(CH₃)-, X₁ et X₂ représentent chacun -P(C₆H₅)₂.

14. Procédé selon l'une ou plusieurs des revendications 1 à 13, **caractérisé en ce qu'**on utilise des complexes du ruthénium du type [(halogénure de Ru)₂(diphosphine de formule VII) (diamine chirale)] ou du type [(halogénure de Ru)₂(ligand de formule XIII)(phosphine tertiaire)] comme catalyseurs d'hydrogénation homogènes et chiraux, où halogénure représente de préférence Cl, Br ou I.

15. Procédé selon l'une ou plusieurs des revendications 1 à 6, **caractérisé en ce que** les trialkylborchydrures de métal alcalin et les trialkylaluminohydrures de métal alcalin correspondent aux formules XXI et XXIa,
métal alcalin[B(R₁₃)₃H] (XXI),
métal alcalin[Al(R₁₃)₃H] (XXIa),
où métal alcalin représente Li, Na ou K et R₁₃ représente un radical alkyle linéaire ou ramifié comprenant 1 à 18, de préférence 3 à 18 et de manière particulièrement préférée 4 à 12 atomes de carbone.

16. Procédé selon la revendication 15, **caractérisé en ce qu'**alkyle est ramifié en position α.

17. Procédé selon la revendication 15 ou 16, **caractérisé en ce qu'**il s'agit, pour R₁₃, d'un radical choisi dans le groupe constitué par but-2-yle, pent-2-yle, hex-2-yle, hept-2-yle, oct-2-yle, 2-diméthylbut-1-yle et 1,2-diméthylbut-1-yle, 2-éthylpent-1-yle, hex-2-yle, 2-méthylhex-1-yle ou 2-éthylhex-1-yle, 1,2,2-triméthyléth-1-yle et 1,2-diméthylbut-1-yle.
